# FASCICULE DE BREVET EUROPEEN

(11) **EP 0 776 881 B1**
(45) Date de publication et mention de la délivrance du brevet: **08.12.1999**
(21) Numéro de dépôt: 96402423.6
(22) Date de dépôt: 13.11.1996
(51) Int. Cl.: C07C 65/17, A61K 31/19, C07C 65/26, C07C 65/28, C07C 271/00, C07D 211/00, C07D 307/00, C07D 333/00, C07D 207/00

(54) **Composés biaromatiques portant un groupement adamantyl en para, compositions pharmaceutiques et cosmétiques les contenant et utilisations**
Bio-aromatische, eine Adamantylgruppe in para-Position enthaltende Verbindungen, diese enthaltende pharmazeutische und kosmetische Zusammensetzungen und ihre Verwendung
Bi-aromatic compounds comprising an adamantyl group in para-position, pharmaceutical and cosmetic compositions containing them and their utilization

(30) Priorité: 01.12.1995 FR 9514261
(43) Date de publication de la demande: 04.06.1997
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: Bernardon, Jean-Michel, 06650 Le Rouret (FR); Charpentier, Bruno, 06410 Biot (FR)
(74) Mandataire: Tezier Herman, Béatrice

(56) Documents cités:
- EP-A- 0 514 264
- EP-A- 0 552 282
- CONGR.INT.TECHNOL.PHARM., 5TH, no. 3, 1989, pages 375-383, XP000578933 JAMOULLE,J.C. ET AL.: "STRUCTURE PERMEATION RELATIONSHIPS OF RETINOID-LIKE SUBSTANCES IN A MODEL BARRIER SYSTEM"

## Description

L'invention concerne, à titre de produits industriels nouveaux et utiles, des composés biaromatiques. Elle concerne également l'utilisation de ces nouveaux composés dans des compositions pharmaceutiques destinées à un usage en médecine humaine ou vétérinaire, ou bien encore dans des compositions cosmétiques.

Dans la demande de brevet EP-A-552282 il a été décrit des composés biaromatiques présentant des activités dans les domaines de la différentiation et de la prolifération cellulaire. Ces composés biaromatiques peuvent présenter une liaison 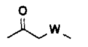 entre les cycles mais ne présentent pas un groupement alkoxy ayant un nombre d'atomes de carbone supérieur à 6 sur le cycle portant éventuellement le groupement adamantyl.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les composés selon l'invention peuvent être représentés par la formule générale (I) suivante : 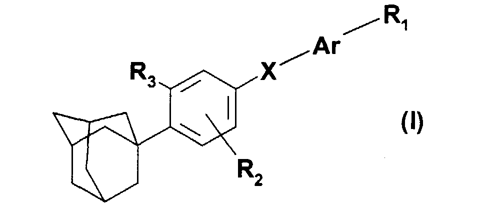 dans laquelle:
- R₁ représente
   (i) le radical -CH₃
   (ii) le radical -(CH₂)n-O-R₄
   (iii) le radical -O-(CH₂)m-(CO)n-R₅
   (iv) le radical -CO-R₆
   (v) le radical -CO-O-R₇
   les valeurs m et n, ainsi que les différents radicaux R₄ à R₇ ayant la signification donnée ci-après,
- R₂ représente un atome d'hydrogène, d'halogène, un radical alkyle linéaire ou ramifié ayant de 1 à 20 atomes de carbone, un radical -OR₄, un radical -O-CH₂-O-CH₂-CH₂-O-CH₃.
- R₃ représente:
   (ii) un radical -Y-(CH₂)p-Y-(CH₂)q-R₈
   (ii) un radical -(CH₂)p-(Y)s-(CH₂)q-R₈
   (iii) un radical -Y-(CH₂)q-R₈
   (v) un radical -CH=CH-(CH₂)r-R₈
   les valeurs p, q, r, s et les radicaux Y et R₈ ayant la signification donnée ci-après,
- X représente les liaisons de formules (a)-(m) suivantes pouvant être lues dans les deux sens : 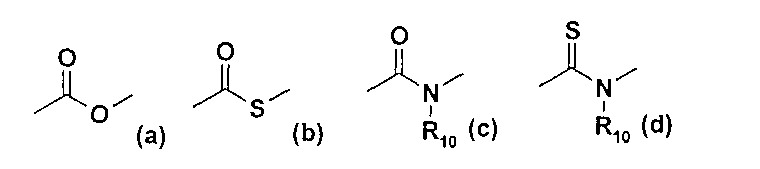 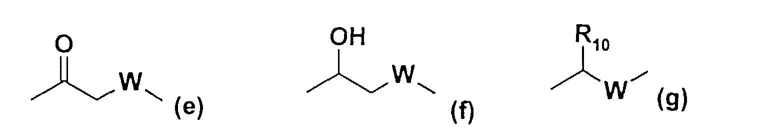 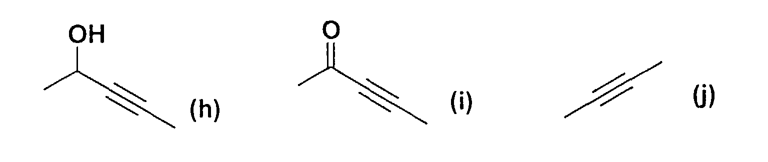 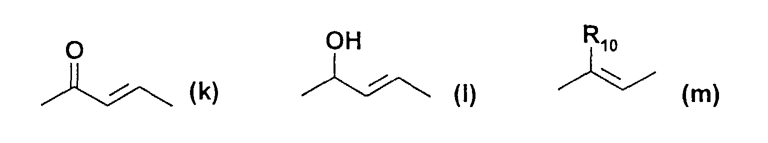
- Ar représente un radical choisi parmi les radicaux de formules (a)-(f) suivantes : 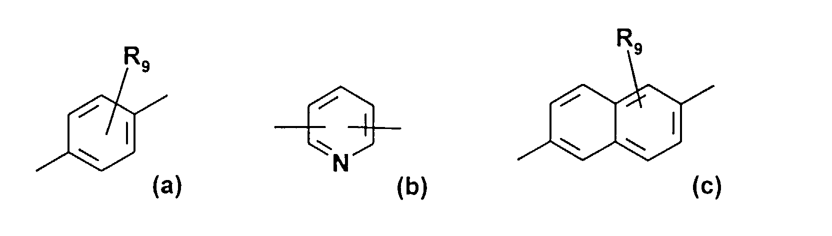 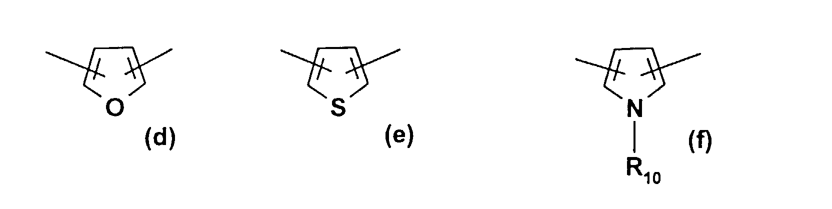 étant entendu que dans tout ce qui précède :
- m est un nombre entier égal à 1,2 ou 3
- n est un nombre entier égal à 0 ou 1
- p est un nombre entier compris inclusivement entre 1 et 12
- q est un nombre entier compris inclusivement entre 0 et 12
- r est un nombre entier compris inclusivement entre 0 et 10.
- s est un nombre entier égal à 0 ou 1
- t est un nombre entier égal à 0, 1 ou 2
- Y représente l'atome d'oxygène ou le radical S(O)t
- W représente l'atome d'oxygène, le radical S(O)t ou le radical N-R₁₀
- R₄ représente un atome d'hydrogène, un radical alkyle inférieur ou un radical -CO-R_{11,}
- R₅ représente un alkyle inférieur ou un hétérocycle,
- R₆ représente un atome d'hydrogène, un radical alkyle inférieur ou un radical 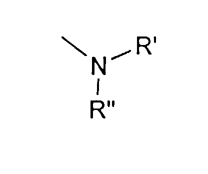 dans lequel R' et R" représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- R₇ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- R8 représente un atome d'hydrogène, un radical alkyle ramifié ayant de 1 à 20 atomes de carbone, un radical cycloaliphatique en C3-C6, un radical monohydroxyalkyle ou un radical polyhydroxyalkyle dont les hydroxy sont éventuellement protégés sous forme de méthoxy, d'acétoxy ou d'acétonide, un radical aryle, un radical alkényle, un radical alkynyle, un radical -CO-R₆, un radical -CO-O-R₇, un radical aminoalkyle dont la fonction amine est éventuellement substituée par un ou deux groupements alkyles inférieurs, un hétérocycle,
- R₉ représente un atome d'hydrogène,d'halogène, un radical allkyle linéaire ou ramifié ayant de 1 à 20 atomes de carbone, un radical -OR₄, un radical -O-CH₂-O-CH₂-CH₂-O-CH₃,
- R₁₀, identique ou différent, représente un atome d'hydrogène, un radical alkyle inférieur,
- R₁₁ représente un radical alkyle inférieur,
étant donné que lorsque X représente la liaison de formule (e) et R₃ représente le radical (iii) dans lequel Y représente un atome d'oxygène et R₈ représente un atome d'hydrogène, alors q doit être supérieur à 6.

L'invention vise également les sels des composés de formule (I) lorsque R₁ ou R₈ représente une fonction acide carboxylique et lorsque R₈ représente une fonction amine, les analogues chiraux et les isomères géométriques desdits composés de formule (I). Lorsque les composés selon l'invention se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique. Lorsque les composés selon l'invention se présentent sous forme de sels par addition d'une base, il s'agit de sels d'un métal alcalin ou alcalino-terreux ou encore de zinc ou d'une amine organique.

Selon la présente invention, on entend par radical alkyle inférieur un radical ayant de 1 à 12, de préférence de 1 à 9, atomes de carbone, avantageusement les radicaux méthyle, éthyle, isopropyle, butyle, tertiobutyle, hexyle, nonyle et dodécyle.

Par radical alkyle linéaire ayant de 1 à 20 atomes de carbone, on entend notamment les radicaux méthyle, éthyle, propyle, 2-éthyl-hexyle, octyle, dodécyle, hexadécyle et octadécyl.

Par radical alkyle ramifié ayant de 1 à 20 atomes de carbone, on entend notamment les radicaux 2-méthylbutyle, 2-méthylpentyle, 1-méthylhexyle, 3-méthylheptyle.

Parmi les radicaux monohydroxyalkyle, on préfère un radical présentant 2 ou 3 atomes de carbone, notamment un radical 2-hydroxyéthyle, 2-hydroxypropyle ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle, on préfère un radical phényle éventuellement substitué par au moins un atome d'halogène, un hydroxyle ou une fonction nitro.

Parmi les radicaux aralkyle, on préfère le radical benzyle ou phénéthyle éventuellement substitué par au moins un atome d'halogène, un hydroxyle ou une fonction nitro.

Parmi les radicaux alkényle, on préfère un radical contenant de 1 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Par radical aminoalkyle, on entend un radical contenant de préférence de 1 à 6 atomes de carbone, notamment les radicaux aminométhyle, 3-aminopropyle, 6-aminohexyle.

Parmi les radicaux alkynyle, on préfère un radical ayant de 2 à 6 atomes de carbone, notamment un radical propargyle.

Parmi les radicaux cycloaliphatiques de 3 à 6 atomes de carbone, on peut citer plus particulièrement le radical cyclopropyle et cyclohexyle.

Lorsque les radicaux R₂ et R₉ représentent un atome d'halogène, celui-ci est de préférence un atome de fluor, de brome ou de chlore.

Parmi les composés de formule (I) ci-dessus rentrant dans le cadre de la présente invention, on peut notamment citer les suivants:
Acide 4-[4-(1-adamantyl)-3-méthoxybenzoyloxy]benzoïque.
Acide (E)-4-[[2-[4-(1-adamantyl)-3-hydroxyphényl]éthényl]]benzoïque.
Acide (E)-4-[[2-[4-(1-adamantyl)-3-propyloxyphényl]éthényl]]benzoïque.
Acide (E)-4-[[2-[4-(1-adamantyl)-3-heptyloxyphényl]éthényl]]benzoïque.
Acide (E)-4-[[2-[4-(1-adamantyl)-3-méthoxyphényl]éthényl]]benzoïque.
Acide 4-[4-(1-adamantyl)-3-méthoxyéthoxyméthoxyphényléthynyl]benzoïque.
Acide (E)-4-[[2-[4-(1-adamantyl)-3-(5-carbamoylpentyloxy)phényl]éthényl]] benzoïque.
Acide (E)-4-[[2-[4-(1-adamantyl)-3-méthoxyphényl]1-propényl]]benzoïque.
Acide (E)-4-[[2-[4-(1-adamantyl)-3-(3-hydroxypropyloxy)phényl]éthényl]] benzoïque.
Acide (E)-4-[[2-[4-(1-adamantyl)-3-(6-hydroxyhexyloxy)phényl]éthényl]] benzoïque.
Acide 4-[[3-oxo-3-[4-(1-adamantyl)-3-methoxyphényl]-1-propenyl]]benzoïque.
Acide 4-[4-(1-adamantyl)-3-methoxyethoxymethoxybenzoylthio]benzoïque.
Acide 4-[4-(1-adamantyl)-3-methoxyethoxymethoxybenzoyloxy]benzoïque.
Acide 4-[4-(1-adamantyl)-3-methoxyethoxymethoxybenzamido]benzoïque.
Acide (E)-4-[2-(4-(1-adamantyl)-3-methoxyethoxymethoxyphenyl)ethenyl] benzoïque.
Acide (E)-4-[2-(4-(1-adamantyl)-3-methoxyethoxymethoxyphenyl)-1-propenyl] benzoïque.
Acide (Z)-4-[2-(4-(1-adamantyl)-3-methoxyethoxymethoxyphenyl)-1-propenyl] benzoïque.
Acide 4-[4-(1-adamantyl)-3-methoxyethoxymethoxybenzoylmethyloxy] benzoïque.
Acide 4-[[3-oxo-3-[4-(1-adamantyl)-3-methoxyethoxymethoxyphényl]-1-propynyl]]benzoïque.
Acide 4-[[3-hydroxy-3-[4-(1-adamantyl)-3-methoxyethoxymethoxyphényl]-1-propynyl]]benzoïque.
Acide (E)4-[[3-oxo-3-[4-(1-adamantyl)-3-methoxyethoxymethoxyphenyl]1-propenyl]]benzoïque.
Acide 4-[[3-hydroxy-3-[4-(1-adamantyl)-3-methoxyethoxymethoxyphenyl]1-propenyl]]benzoïque.
4-[4-(1-adamantyl)-3-methoxyethoxymethoxythiobenzamido]benzoate d'allyle.
Acide 4-[[3-hydroxy-3-[4-(1-adamantyl)-3-methoxyphenyl]-1-propynyl]]benzoïque.
Acide 2-hydroxy-4-[[3-hydroxy-3-[4-(1-adamantyl)-3-methoxyphenyl]-1-propynyl]]benzoïque.
Acide 2-hydroxy-4-[[3-hydroxy-3-[4-(1-adamantyl)-3-methoxyethoxymethoxy phenyl]-1-propynyl]]benzoïque.
4-[[3-hyd roxy-3-[4-(1-adamantyl)-3-methoxyethoxymethoxyphenyl]-1-propynyl]]benzaldehyde.
4-[[3-hydroxy-3-[4-(1-adamantyl)-3-methoxyethoxymethoxyphenyl]1-propynyl]]benzenemethanol.
N-ethyl-4-[4-(N-ethyl-4-[4-(1-adamantyl)-3-méthoxyéthoxyméthoxyphényléthynyl]benzamide.
N-(4-hydroxyphenyl)-4-[4-(1-adamantyl)-3-méthoxyéthoxyméthoxyphényl éthynyl]benzamide.
4-[4-(1-adamantyl)-3-méthoxyéthoxyméthoxyphényléthynyl]phenol.

Selon la présente invention les composés de formule (I) plus particulièrement préférés sont ceux pour lesquels au moins l'une des, et de préférence toutes les, conditions suivantes est remplie :
- R₁ est le radical

   -COO-R₇ et -CO-R₆.
- R₃ est le radical

   -Y-(CH₂)p-Y-(CH₂)q-R₈.

   -(CH₂)p-(Y)n-(CH₂)q-R₈.

   -Y-(CH₂)q-R₈.
- X représente la liaison de formule (a), (h), (i), (j), (k) ou (m).
- Ar représente le radical de formule (a) ou (b).

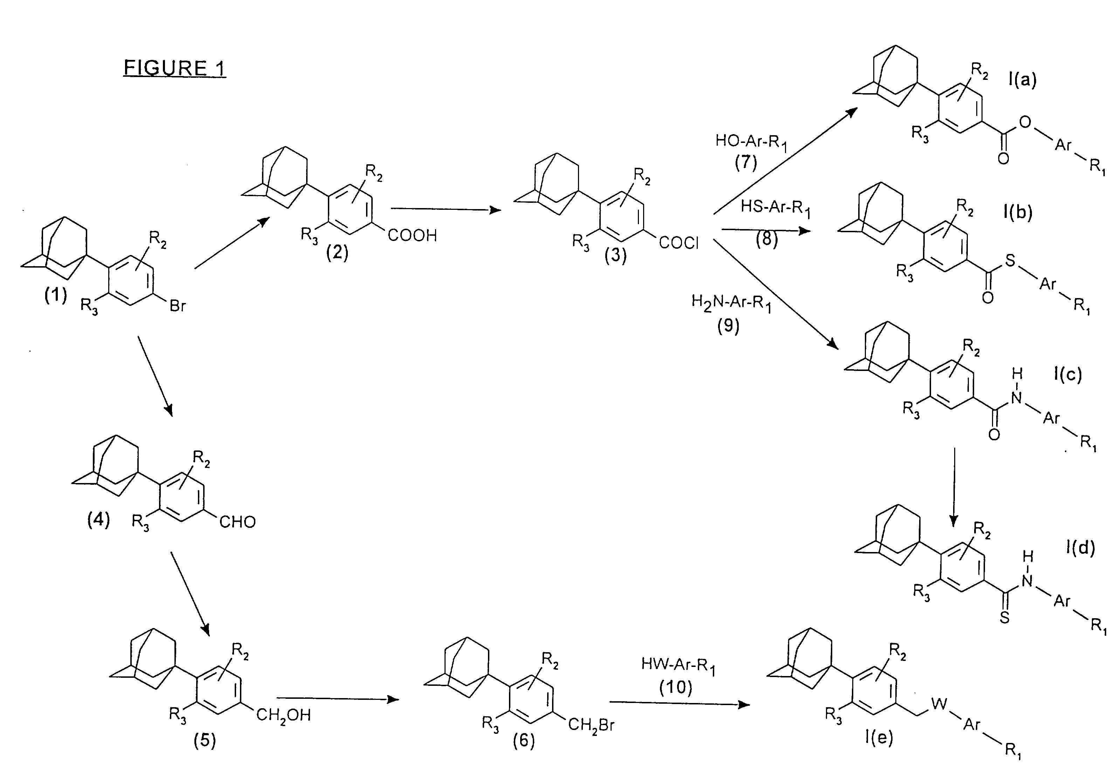

La présente invention a également pour objet les procédés de préparation des composés de formule (I), en particulier selon les schémas réactionnels donnés aux figures 1, 2, 3 et 4.

Ainsi les composés de formule I(a) peuvent être obtenus (figure 1) en faisant réagir en milieu anhydre, dans un solvant organique de préférence le THF et en présence d'une amine tertiaire (par exemple triéthylamine ou pyridine) une forme activée d'un acide benzoïque (2) par exemple un chlorure d'acide (3) sur un composé phénolique de formule (7).

Ainsi les composés de formule I(b) peuvent être obtenus (figure 1) en faisant réagir en milieu anhydre, dans un solvant organique de préférence le THF et en présence d'une amine tertiaire (par exemple triéthylamine ou pyridine) une forme activée d'un acide benzoïque (2) par exemple un chlorure d'acide (3) sur un composé thiophénolique de formule (8).

Ainsi les composés de formule I(c) peuvent être obtenus (figure 1) en faisant réagir en milieu anhydre, dans un solvant organique de préférence le THF et en présence d'une amine tertiaire (par exemple triéthylamine ou pyridine) une forme activée d'un acide carboxylique aromatique (2) par exemple un chlorure d'acide (3) sur un composé aminé de formule (9).

Ainsi les composés de formule I(d) peuvent être obtenus (figure 1) à partir des composés de formule 1(c) par réaction avec le réactif de Lawesson.

Ainsi les composés de formule 1(e) peuvent être préparés (figure 1) à partir des alcools benzyliques (5) par transformation en dérivés bromés (6) avec du tribromure de phosphore puis réaction en présence de carbonate de potassium ou d'un hydrure alcalin (hydrure de sodium) ou par transfert de phase en utilisant par exemple le bromure de tétrabutylammonium comme sel quaternaire avec un composé (10) porteur d'une fonction hydroxy ou thiol ou amino.

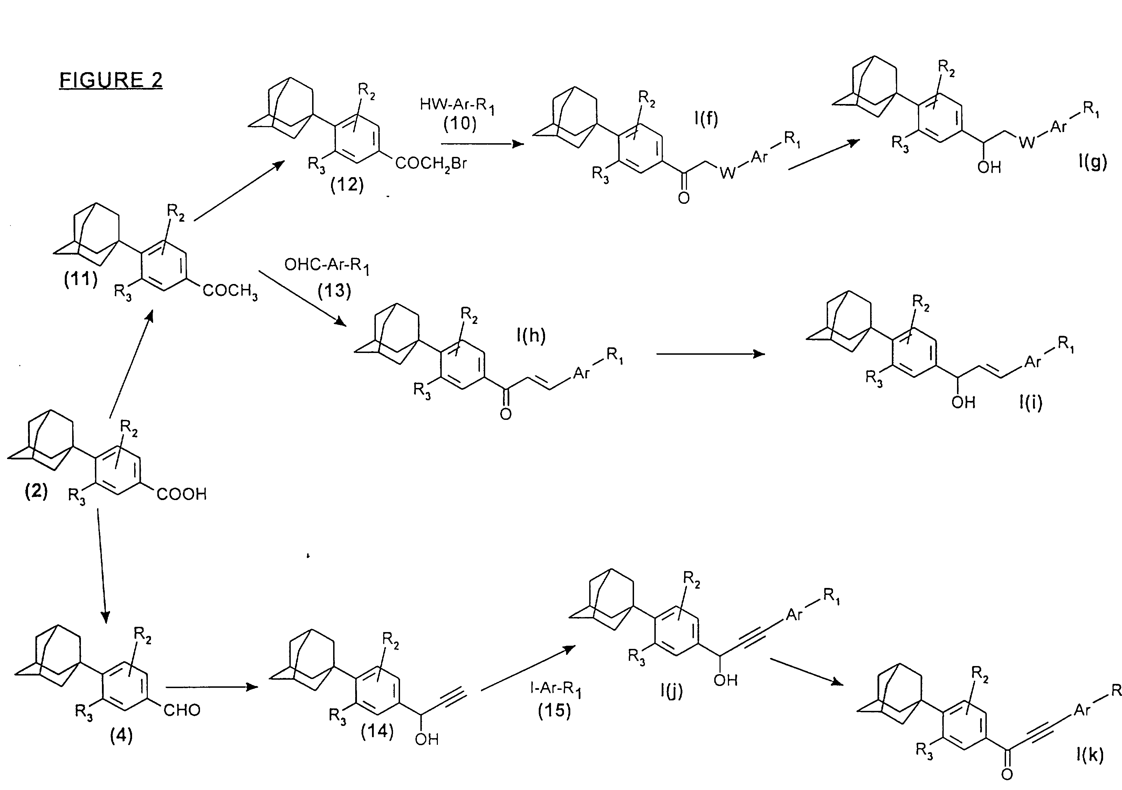

Ainsi les composés de formule I(f) peuvent être obtenus (figure 2) à partir des dérivés acetophenones (11) par transformation en dérivé bromé (12) à l'aide du brome puis réaction en présence de carbonate de potassium ou d'un hydrure alcalin (hydrure de sodium) ou par transfert de phase en utilisant par exemple le bromure de tétrabutylammonium comme sel quaternaire avec un composé (10) porteur d'une fonction hydroxy ou thiol ou amino. Les composés de formule I(g) peuvent être obtenus à partir du dérivé I(f) par réaction avec du borohydrure de sodium dans un solvant alcoolique.

Ainsi les composés de formule I(h) peuvent être obtenus (figure 2) à partir des dérivés acetophenones (11) par réaction avec des dérives aldéhydes aromatiques (13) en présence de méthylate de sodium ou de soude dans un solvant alcoolique tel le méthanol. A partir de ces composés par réaction avec du borohydrure de sodium en présence de trichlorure de Cerium, on obtient les composés de formule I(i).

Ainsi les composés de formule I(j) peuvent être préparés (figure 2) à partir des dérives aldéhydes aromatiques (4) par réaction avec le triméthylsilylacétylénure de lithium puis déprotection avec le fluorure de tétrabutylammonium pour obtenir les dérivés α-hydroxyacétyléniques (14). Puis couplage avec les dérivés halogénés (15) de préférence iodé en présence d'un catalyseur au Palladium (par exemple le chlorure de Bis-(triphénylphosphine) palladium(ll) dans un solvant tel que la triéthylamine. L'oxydation de ces composés avec soit le pyridinium dichromate ou l'oxyde de manganèse ou le réactif de Swern conduit aux dérivés de formule I(k).

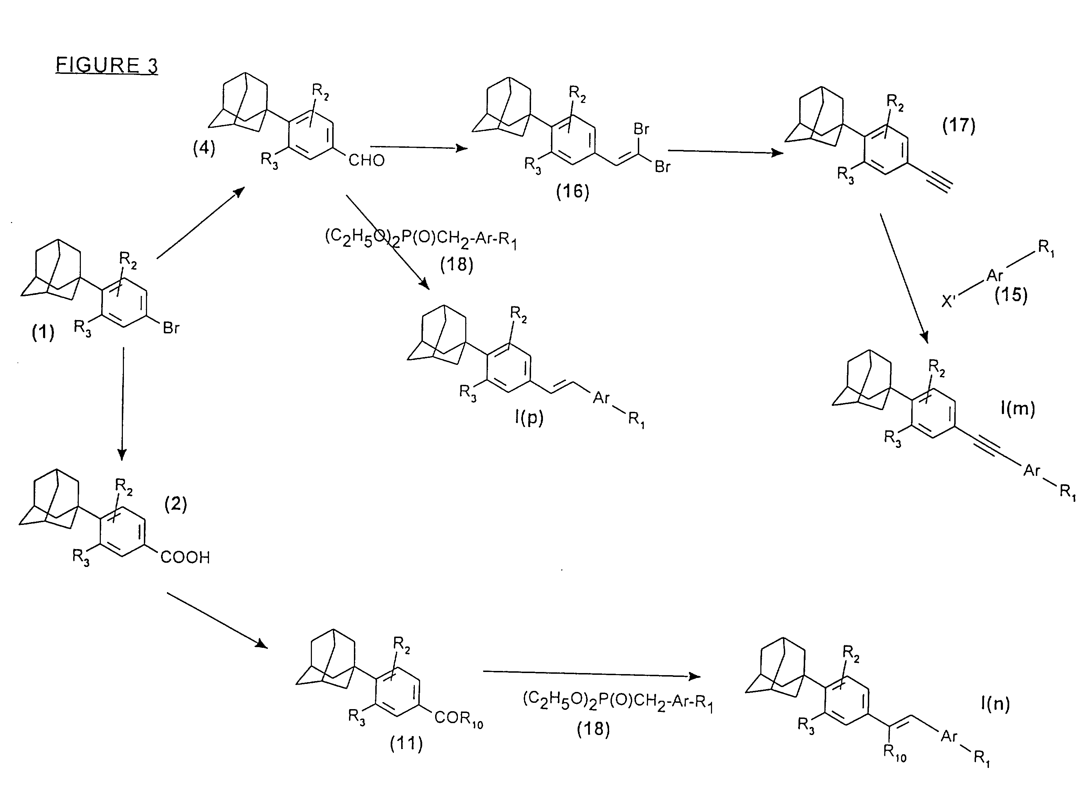

Ainsi, les composés de formule I(m) peuvent être préparés (figure 3) à partir des dérives aldéhydes aromatiques (4) par réaction avec le tétrabromure de carbone et la triphénylphosphine pour donner des dérivés 2',2'-dibromostyrènes (16) qui sont ensuite transformés en dérivés acétyléniques (17) par action du n-butyl lithium dans un solvant tel le THF. Puis couplage avec les dérivés halogénés (15) de préférence iodés en présence d'un catalyseur au Palladium (par exemple le chlorure de Bis-(triphénylphosphine)palladium(ll) dans un solvant tel la triéthylamine.

Ainsi les composés de formule I(p) et I(n) peuvent être préparés (figure 3) à partir respectivement des dérives aldéhydes aromatiques (4) et des dérivés acetophenones (11) suivant une réaction de type Horner-Emmons ou Wittig avec des dérivés phosphonates aromatiques (18) ou des dérivés phosphines aromatiques.

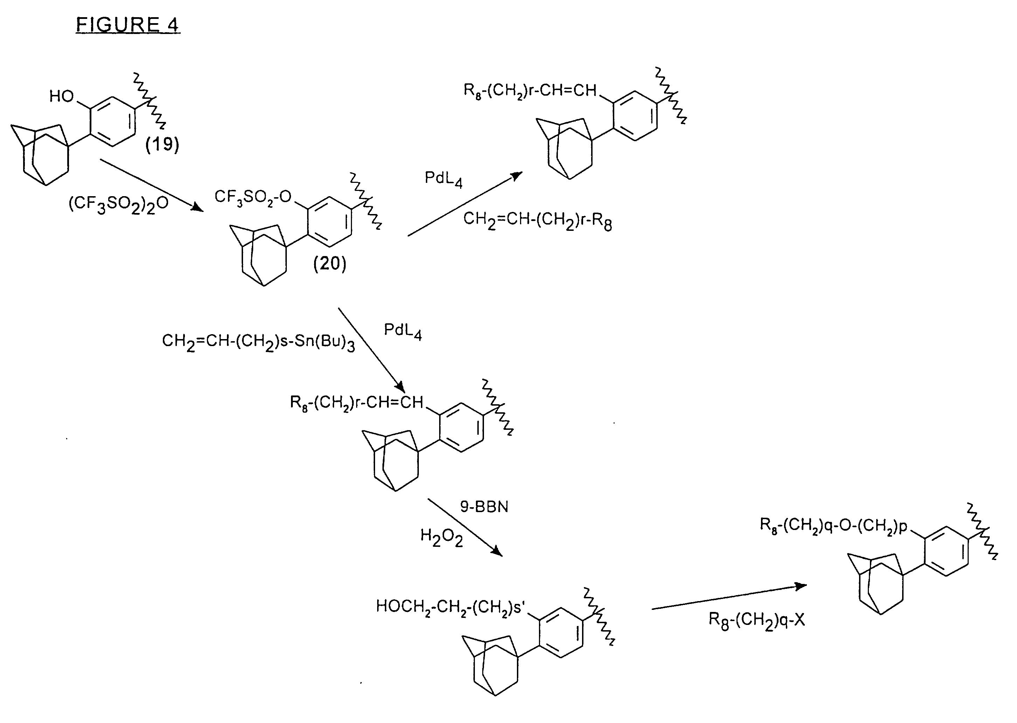

Lorsque R₃ représente les radicaux -(CH₂)p-(Y)n-(CH₂)q-R₈ ou -CH=CH-(CH₂)r-R₈, les composés peuvent être obtenus (figure 4 dans laquelle s' égal p-2) à partir des dérivés phénoliques (19) que l'on transforme en dérivés triflates (20) puis substitution nucléophile en présence d'un catalyseur au palladium selon les conditions générales décrites par:
- S. Cacchi et al. Tetrahedron Letter, 1986, 27, 3931-3934.
- W. J. Scott et al. J. Org. Chem. 1985, 50, 2302-2308.
- J. K. Stille et al. J. Am. Chem. Soc; 1987, 109, 5478-5486.

Lorsque R₁ représente le radical -COOH, les composés sont préparés en protégeant R₁ par un groupe protecteur de type alkyle, allylique, benzylique ou tert-butylique.

Le passage à la forme libre peut être éffectué:
- dans le cas d'un groupe protecteur alkyle, au moyen de soude ou d'hydroxyde de lithium dans un solvant alcoolique tel le méthanol ou dans le THF.
- dans le cas d'un groupe protecteur allylique, au moyen d'un catalyseur tel certains complexes de métaux de transition en présence d'une amine secondaire telle la morpholine.
- dans le cas d'un groupement protecteur benzylique, par débenzylation en présence d'hydrogène au moyen d'un catalyseur tel que le palladium sur charbon.
- dans le cas d'un groupement protecteur de type tert-butylique au moyen d'iodure de triméthylsilyle.

La présente invention a également pour objet à titre de médicament les composés de formule (I) telle que définie ci-dessus.

Ces composés présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités de ces composés respectivement dans les domaines de la différenciation et de la prolifération cellulaire. Dans le test de différenciation des cellules (F9), il est possible d'évaluer une activité agoniste, comme une activité antagoniste aux récepteurs de l'acide rétinoïque. En effet, un antagoniste est inactif lorsqu'il est seul dans ce test, mais inhibe partiellement ou totalement l'effet produit par un rétinoïde agoniste sur la morphologie et sur la sécrétion de l'activateur plasminogène. Certains de ces composés présentent donc également une activité dans un test qui consiste à identifier des molécules antagonistes des RARs, tel que décrit dans la demande de brevet français n° 95-07302 déposée le 19 juin 1995 par la Demanderesse. Ce test comprend les étapes suivantes : (i) on applique topiquement sur une partie de la peau d'un mammifère une quantité suffisante d'une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse sur la partie de la peau ainsi traitée du mammifère. Ainsi, la réponse à une application topique sur l'oreille d'un mammifère d'une molécule agoniste des RARs qui correspond à une augmentation de l'épaisseur de cette oreille peut être inhibée par l'administration par voie systémique ou topique d'une molécule antagoniste des RARs.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphénylimidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-triynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### EXEMPLE 1

### Acide 4-[-4-(1-adamantyl)-3-méthoxybenzoyloxy]benzoïque

### (a) 2-(1-adamantyl)-5-bromophénol.

Dans un ballon on introduit 17 g (0,1 mole) de 3-bromophénol 15,2 g (0,1 mole) de 1-adamantanol et 50 ml de dichlorométhane. On ajoute goutte à goutte 5 ml d'acide sulfurique concentré et agite à température ambiante 24 heures. On verse le milieu réactionnel dans l'eau glacée, neutralise avec du bicarbonate de sodium, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (50-50). On recueille 15,2 g (50%) de phénol attendu de point de fusion 112-4°C.

### (b) 4-(1-adamantyl)-1-bromo-3-méthoxybenzène.

Dans un tricol, on introduit 1,6 g (53 mmoles) d'hydrure de sodium (80% dans l'huile) et 50 ml de DMF. On ajoute goutte à goutte une solution de 15 g (49 mmoles) de 2-(1-adamantyl)-5-bromophénol dans 100 ml de DMF et agite jusqu'à cessation du dégagement gazeux. On ajoute ensuite tout en refroidissant avec un bain de glace 3 ml (49 mmoles) d'iodure de méthyle et agite à température ambiante 6 heures. On verse le milieu réactionnel dans l'eau, extrait avec de l'éther éthylique, décante la phase organique, sèche sur sulfate de magnésium, évapore. Le résidu obtenu est trituré dans l'hexane, filtré, séché. on recueille 12,6 g (80%) du produit attendu de point de fusion 155-60°C.

### (c) acide 4-(1-adamantyl)-3-méthoxybenzoïque.

Le composé précédent (11,3 g 35 mmoles)) est dissous dans 200 ml de THF. La solution obtenue est ajoutée goutte à goutte sur du magnésium (1,3 g 52,5 mmoles) et un cristal d'iode. Après introduction, on chauffe à reflux pendant deux heures, refroidit à -78°C et fait passer un courant de CO₂ pendant une heure. On laisse remonter à température ambiante, verse le milieu réactionnel dans une solution aqueuse saturée en chlorure d'ammonium, extrait avec de l'éther éthylique, décante la phase organique, sèche sur sulfate de magnésium, évapore. Le résidu obtenu est purifié par chromatographie sur colonne de silice élué avec un mélange de dichlorométhane et d'éther éthylique (90-10). On recueille 5,1 g (51%) de l'acide attendu de point de fusion 259-60°C.

### (d) chlorure de 4-(1-adamantyl)-3-méthoxybenzoyle.

Dans un ballon on introduit 2,1 g (7,34 mmoles) d'acide 4-(1-adamantyl)-3-méthoxybenzoïque et 20 ml de chlorure de thionyle et chauffe à reflux jusqu'à cessation du dégagement gazeux. On évapore à sec le milieu réactionnel et recueille 2,2 g (100%) de chlorure d'acide qui sera utilisé tel quel pour la suite de la synthèse.

### (e) 4-[-4-(1-adamantyl)-3-méthoxybenzoyloxy]benzoate de benzyle.

Dans un ballon on introduit 1,67 g (7,34 mmoles) de 4-hydroxybenzoate de benzyle 1 ml (7,34 mmoles) de triéthylamine et 40 ml de THF. On ajoute goutte à goutte une solution de 2,2 g (7,34 mmoles) de chlorure de 4-(1-adamantyl)-3-méthoxybenzoyle et agite à température ambiante pendant 8 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (60-40). Après évaporation des solvants, on recueille 3,1 g (85%) de l'ester benzylique attendu de point de fusion 128-9°C.

### (f) acide 4-[-4-(1-adamantyl)-3-méthoxybenzoyloxy]benzoïque.

Dans un réacteur, on introduit 1,8 g (3,6 mmoles) de 4-[-4-(1-adamantyl)-3-méthoxybenzoyloxy]benzoate de benzyle et 50 ml de dioxanne. On introduit après avoir purgé à l'azote 1 g de Pd sur charbon (10%) puis de l'hydrogène sous une pression de 7,5 bars. On agite à température ambiante pendant 3 heures, filtre le catalyseur, évapore le filtrat. Le résidu obtenu est trituré dans 40 ml d'éther éthylique, filtré. On recueille 1,1 g (71%) de l'acide attendu de point de fusion 273-4°C.

### EXEMPLE 2

### Acide (E)-4-[[2-[4-(1-adamantyl)-3-hydroxyphényl]éthényl]]benzoïque.

### (a) 4-(1-adamantyl)-1-bromo-3-tert-butyldiméthylsilyloxybenzène.

Dans un ballon, on introduit sucessivement 15,36 g (50 mmoles) de 2-(1-adamantyl)-5-bromophénol 150 ml de DMF 7,7 ml (55 mmoles) de triéthylamine 305 mg de 4-diméthylaminopyridine et ajoute une solution de 8,3 g ((55 mmoles) de chlorure de tert-butyldiméthylsilane et agite à température ambiante pendant 12 heures. On verse le milieu réactionnel dans l'eau, extrait avec de l'éther éthylique, décante la phase organique, sèche sur sulfate de magnésium, évapore. Le solide obtenu est recristallisé dans l'acétone, on recueille 19,9 g (94%) de produit attendu de point de fusion 86-8°C.

### (b) 4-(1-adamantyl)-3-tert-butyldiméthylsilyloxybenzènecarboxaldéhyde.

De manière analogue à l'exemple 6(a) à partir de 19,8 g (47 mmoles) de 4-(1-adamantyl)-1-bromo-3-tert-butyldiméthylsilyloxybenzène, on obtient 14,8 g (85%) de l'aldéhyde attendu de point de fusion 114-6°C.

### (c) (E)-4-[[2-[4-(1-adamantyl)-3-hydroxyphényl]éthényl]]benzoate d'éthyle.

Dans un tricol et sous courant d'azote, on introduit 724 mg (24 mmoles) d'hydrure de sodium (80% dans l'huile) et 50 ml de THF. On ajoute goutte à goutte une solution composée de 7,4 g (20 mmoles) de 4-(1-adamantyl)-3-tert-butyldiméthylsilyloxybenzènecarboxaldéhyde 7,2 g (24 mmoles) de 4-éthoxycarbonylbenzylphosphonate de diéthyle et 885 mg de 12-crown-6 dans 150 ml de THF et agite à température ambiante pendant 8 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange d'acétate d'éthyle et d'heptane (5-95). Après évaporation des solvants, on recueille 4,8 g (46%) de (E)-4-[[2-[4-(1-adamantyl)-3-tert-butyldiméthylsilyloxyphényl]éthényl]]benzoate d'éthyle de point de fusion 140-1°C et 2,22 g (28%) de (E)-4-[[2-[4-(1-adamantyl)-3-hydroxyphényl]éthényl]]benzoate d'éthyle de point de fusion 228-9°C.

### (d) acide (E)-4-[[2-[4-(1-adamantyl)-3-hydroxyphényl]éthényl]]benzoïque.

Dans un ballon, on introduit 258 mg (0,5 mmole)de (E)-4-[[2-[4-(1-adamantyl)-3-hydroxyphényl]éthényl]]benzoate d'éthyle 105 mg (2,5 mmoles) d'hydroxyde de lithium et 10 ml de THF. On agite à température ambiante pendant 12 heures, verse le milieu réactionnel dans l'eau, neutralise avec de l'acide chlorhydrique, extrait avec de l'éther éthylique, décante la phase organique, sèche sur sulfate de magnésium, évapore. Le résidu obtenu est trituré dans un mélange de dichlorométhane et d'hexane, filtré et séché. On recueille 95 mg (51%) de l'acide attendu de point de fusion 334-5°C.

### EXEMPLE 3

### Acide (E)-4-[[2-[4-(1-adamantyl)-3-propyloxyphényl]éthényl]]benzoïque.

### (a) (E)-4-[[2-[4-(1-adamantyl)-3-propyloxyphényl]éthényl]]benzoate d'éthyle.

Dans un ballon, on introduit 66 mg (2,2 mmoles) d'hydrure de sodium (80% dans l'huile) et 10 ml de DMF. On ajoute goutte à goutte une solution de 805 mg (2 mmoles) de (E)-4-[[2-[4-(1-adamantyl)-3-hydroxyphényl]éthényl]]benzoate d'éthyle dans 20 ml de DMF et agite jusqu'à cessation du dégagement gazeux. On ajoute ensuite 200 µl (2,2 mmoles) de 1-bromopropane et agite à température ambiante pendant 8 heures, verse le milieu réactionnel dans l'eau, extrait avec de l'éther éthylique, décante la phase organique, sèche sur sulfate de magnésium, évapore. Le résidu obtenu est trituré dans l'heptane, filtré séché. On recueille 826 mg (93%) de l'ester éthylique attendu de point de fusion 143-4°C.

### (b) acide (E)-4-[[2-[4-(1-adamantyl)-3-propyloxyphényl]éthényl]]benzoïque.

De manière analogue à l'exemple 2(d) à partir de 818 mg (1,84 mmoles) de l'ester éthylique précédent, on obtient 390 mg (51%) de l'acide attendu de point de fusion 291-3°C.

### EXEMPLE 4

### Acide (E)-4-[[2-[4-(1-adamantyl)-3-heptyloxyphényl]éthényl]]benzoïque.

### (a) (E)-4-[[2-[4-(1-adamantyl)-3-heptyloxyphényl]éthényl]]benzoate d'éthyle.

De manière analogue à l'exemple 3(a) par réaction de 805 mg (2 mmoles) de (E)-4-[[2-[4-(1-adamantyl)-3-hydroxyphényl]éthényl]]benzoate d'éthyle avec 346 µl (2,2 mmoles) de 1-bromoheptane, on obtient 872 mg (87%) de l'ester éthylique attendu de point de fusion 123-4°C.

### (b) acide (E)-4-[[2-[4-(1-adamantyl)-3-heptyloxyphényl]éthényl]]benzoïque.

De manière analogue à l'exemple 2(d) à partir de 865 mg (1,73 mmoles) de l'ester éthylique précédent, on obtient 620 mg (76%) de l'acide attendu de point de fusion 262-3°C.

### EXEMPLE 5

### Acide (E)-4-[[2-[4-(1-adamantyl)-3-méthoxyphényl]éthényl]]benzoïque.

### (a) (E)-4-[[2-[4-(1-adamantyl)-3-méthoxyphényl]éthényl]]benzoate d'éthyle.

De manière analogue à l'exemple 3(a) par réaction de 805 mg (2 mmoles) de (E)4-[[2-[4-(1-adamantyl)-3-hydroxyphényl]éthényl]]benzoate d'éthyle avec 137 µl (2,2 mmoles) d'iodure de méthyle, on obtient 768 mg (92%) de l'ester éthylique attendu de point de fusion 152-3°C.

### (b) acide (E)4-[[2-[4-(1-adamantyl)-3-méthoxyphényl]éthényl]]benzoïque.

De manière analogue à l'exemple 2(d) à partir de 763 mg (1,83 mmole) de l'ester éthylique précédent, on obtient 615 mg (87%) de l'acide attendu de point de fusion 284-6°C.

### EXEMPLE 6

### Acide 4-[[2-(4-(1-adamantyl)-3-méthoxyéthoxyméthoxyphényl]éthynyl]] benzoïque.

### (a) 4-(1-adamantyl)-3-méthoxyéthoxyméthoxyphénylcarboxaldéhyde.

Dans un tricol et sous courant d'azote, on introduit 18 g (45,5 mmoles) de 4-(1-adamantyl)-1-bromo-3-méthoxyéthoxyméthoxybenzène et 100 ml de THF. A - 78°C on ajoute goutte à goutte 20 ml d'une solution de n-butyllithium (2,5 M dans l'hexane) et agite 30'. On ajoute ensuite goutte à goutte 3,5 ml de DMF et laisse remonter à température ambiante. On verse le milieu réactionnel dans une solution aqueuse de chlorure d'ammonium, extrait avec de l'éther éthylique, décante la phase organique, sèche sur sulfate de magnésium, évapore. Le résidu obtenu est purifié par chromatographie sur colonne de silice élué avec un mélange de dichlorométhane et d'heptane (30-70). Après évaporation des solvants, on recueille 12,7 g (81%) d'aldéhyde attendu de point de fusion 87-9°C.

### (b) 2',2'-dibromo-4-(1-adamantyl)-3-méthoxyéthoxyméthoxystyrène.

Dans un ballon on introduit 5 g (14,5 mmoles) de 4-(1-adamantyl)-3-méthoxyéthoxyméthoxyphénylcarboxaldéhyde et 30 ml de dichlorométhane. On ajoute successivement 9,6 g (29 mmoles) de tétrabromure de carbone 7,6 g (29 mmoles) de triphénylphosphine et 1,9 g (29 mmoles) de poudre de zinc et agite à température ambiante pendant deux heures. On évapore le milieu réactionnel et purifie le résidu obtenu par chromatographie sur colonne de silice élué avec un mélange de dichlorométhane et d'heptane (40-60). On recueille 3 g (40%) du produit attendu.

### (c) 4-(1-adamantyl)-3-méthoxyéthoxyméthoxyphénylacétylène.

Dans un tricol et sous courant d'azote on introduit 3 g (6 mmoles) de 2',2'-dibromo-4-(1-adamantyl)-3-méthoxyéthoxyméthoxystyrène et 50 ml de THF. A-78°C on ajoute goutte à goutte 4,8 ml (12 mmoles) d'une solution de n-butyllithium (2,5 M dans l'hexane) et laisse remonter à température ambiante une heure. On verse le milieu réactionnel dans l'eau, extrait avec de l'éther éthylique, décante la phase organique, sèche sur sulfate de magnésium, évapore. Le résidu est trituré dans l'heptane, filtré, séché. On recueille 1,5 g (73%) du dérivé acétylénique attendu de point de fusion 86-8°C.

### (d) 4-[[2-[4-(1-adamantyl)-3-méthoxyéthoxyméthoxyphényl]ethynyl]]benzoate de méthyle.

Dans un tricol, on introduit 1,3 g (3,8 mmoles) de 4-(1-adamantyl)-3-méthoxyéthoxyméthoxyphénylacétylène 1 g (3,8 mmoles) de 4-iodobenzoate de méthyle et 15 ml de triéthylamine. On dégaze le milieu réactionnel avec de l'azote pendant 15' et ajoute 100 mg d'iodure de cuivre et 260 mg (0,37 mmole) de chlorure de bis(triphénylphosphine)palladium(ll) et agite à température pendant douze heures. On évapore à sec le milieu réactionnel, reprend par l'eau et l'éther éthylique, décante la phase organique, sèche sur sulfate de magnésium, évapore. Le résidu obtenu est purifié par chromatographie sur colonne de silice élué avec un mélange de dichlorométhane et d'heptane (20-80). Aprés évaporation des solvants, on recueille 1,3 g (72%) de l'ester méthylique attendu.

### (e) acide 4-[[2-[4-(1-adamantyl)-3-méthoxyéthoxyméthoxyphényl]éthynyl]]benzoïque.

De manière analogue à l'exemple 2(d) à partir de 400 mg (0,8 mmole) de l'ester méthylique précédent, on recueille 250 mg (64%) de l'acide attendu de point de fusion 215-7°C.

### EXEMPLE 7

### Acide (E)-4-[[2-[4-(1-adamantyl)-3-(5-carbamoylpentyloxy)phényl] éthényl]]benzoïque.

### (a) (E)-4-[[2-[4-(1-adamantyl)-3-(5-carbamoylpentyloxy)phényl]éthényl]]benzoate d'éthyle.

Dans un ballon, on introduit 805 mg (2 mmoles) de (E)-4-[[2-[4-(1-adamantyl)-3-hydroxyphényl]éthényl]]benzoate d'éthyle 388 mg (2 mmoles) de 6-bromo hexylamide 331 mg de carbonate de potassium et 30 ml de DMF. On chauffe à 70°C pendant 3 jours, verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange d'acétate d'éthyle et d'heptane (80-20). Après évaporation des solvants, on recueille 586 mg (57%) de l'ester éthylique attendu de point de fusion 160-1°C.

### (b) acide (E)-4-[[2-[4-(1-adamantyl)-3-(5-carbamoylpentyloxy)phényl]éthényl]] benzoïque.

Dans un ballon, on introduit 575 mg (1,1 mole) de l'ester éthylique précédent et 20 ml d'éthanol. On ajoute 483 mg (12 mmoles) de soude et chauffe à 40°C pendant 2 heures. On verse le milieu réactionnel dans l'eau acidifie à pH 1 avec de l'acide chlorhydrique, extrait avec de l'acétate d'éthyle, décante la phase organique, sèche sur sulfate de magnésium, évapore. Le solide obtenu est trituré dans le minimum d'éther éthylique, filtré, séché. On recueille 465 mg (88%) de l'acide attendu de point de fusion 261-2°C.

### EXEMPLE 8

### Acide (E)-4-[[2-(4-(1-adamantyl)-3-méthoxyphényl]1-propényl]]benzoïque.

### (a) 4-(1-adamantyl)-3-méthoxyacétophénone.

Dans un tricol et sous courant d'azote on introduit 7,3 g (25,5 mmoles) d'acide 4-(1-adamantyl)-3-méthoxybenzoïque et 300 ml d'éther éthylique anhydre. A -20°C, on ajoute goutte à goutte 32 ml (51 mmoles) de méthyllithium (1,6 M dans l'éther) puis agite pendant trois heures à température ambiante. On verse le milieu réactionnel dans une solution aqueuse saturée en chlorure d'ammonium, décante la phase organique, sèche sur sulfate de magnésium, évapore. On triture le solide obtenu dans l'hexane, filtre, sèche. On recueille 5,05 g (70%) de l'acetophenone attendu de point de fusion 101-2°C.

### (b) acide (E)-4-[[2-[4-(1-adamantyl)-3-méthoxyphényl]1-propényl]]benzoïque.

De manière analogue à l'exemple 2(c) par réaction de 1,9 g (6,6 mmoles) de 4-(1-adamantyl)-3-méthoxyacétophénone avec 2,2 g (7,33 mmoles) de 4-éthoxy carbonylbenzylphosphonate de diéthyle, on obtient 112 mg d'acide (E)-4-[[2-[4-(1-adamantyl)-3-méthoxyphényl]1-propényl]]benzoïque de point de fusion 243-5°C.

### EXEMPLE 9

### Acide (E)-4-[[2-[4-(1-adamantyl)-3-(3-hydroxypropyloxy)phényl]éthényl]] benzoïque.

### (a) (E)-4-[[2-[4-(1-adamantyl)-3-(3-hydroxypropyloxy)phényl]éthényl]]benzoate de méthyle.

De manière analogue à l'exemple 7(a) par réaction de 1 g (2,6 mmoles) de (E)-4-[[2-[4-(1-adamantyl)-3-hydroxyphényl]éthényl]]benzoate de méthyle avec 537 mg (3,8 mmoles) de 3-bromopropanol, on obtient 472 mg (41%) de l'ester méthylique attendu de point de fusion 163-5°C.

### (b) acide (E)-4-[[2-[4-(1-adamantyl)-3-(3-hydroxypropyloxy)phényl]éthényl]] benzoïque.

De manière analogue à l'exemple 7(b) à partir de 464 mg (1,04 mmole) de l'ester méthylique précédent, on obtient 364 mg (84%) de l'acide attendu de point de fusion 268-70°C.

### EXEMPLE 10

### Acide (E)-4-[[2-[4-(1-adamantyl)-3-(6-hydroxyhexyloxy)phényl]éthényl]] benzoïque.

### (a) (E)-4-[[2-[4-(1-adamantyl)-3-(6-hydroxyhexyloxy)phényl]éthényl]]benzoate d'éthyle.

De manière analogue à l'exemple 7(a) par réaction de 1 g (2,5 mmoles) de (E)-4-[[2-[4-(1-adamantyl)-3-hydroxyphényl]éthényl]]benzoate de méthyle avec 710 mg (3,8 mmoles) de 6-bromohexanol, on obtient 1,12 g (89%) de l'ester méthylique attendu de point de fusion 105-7°C.

### (b) acide (E)-4-[[2-[4-(1-adamantyl)-3-(6-hydroxyhexyloxy)phényl]éthényl]] benzoïque.

De manière analogue à l'exemple 7(b) à partir de 1,09 g (2,23 mmoles) de l'ester méthylique précédent, on obtient 943 mg (89%) de l'acide attendu de point de fusion 238-40°C.

### EXEMPLE 11

### Acide 4-[[3-oxo-3-[4-(1-adamantyl)-3-methoxyphényl]-1-propenyl]]benzoïque.

### (a) 2-(1-adamantyl)-5-bromophenol.

Dans un ballon, on introduit 41 g (0,237 mole) de 3-bromophenol 38 g (0,25 mole) de 1-adamantanol et 500 ml de dichloromethane. On ajoute 12,5 ml d'acide sulfurique concentré et agite à température ambiante pendant huit heures. On verse le milieu réactionnel dans l'eau, décante la phase organique, lave à l'eau, sèche sur sulfate de magnésium, évapore. Le résidu obtenu est purifié par chromatographie sur colonne de silice élué avec du dichlorométhane. Après évaporation des solvants, on recueille 45 g (62%) du phenol attendu de point de fusion 112-4°C.

### (b) 2-(1-adamantyl)-5-bromoanisole.

Dans un tricol et sous courant d'azote, on introduit 20 g (65 mmoles) de 2-(1-adamantyl)-5-bromophenol et 200 ml de DMF. On ajoute par petites quantites 1,9 g (65 mmoles) d'hydrure de sodium (80% dans l'huile) et agite jusqu'à cessation du dégagement gazeux. On ajoute ensuite 5,3 ml (84,5 mmoles) de iodomethane et agite à température ambiante trois heures.On verse le milieu réactionnel dans l'eau, extrait avec de l'éther éthylique, décante la phase organique, lave à l'eau, sèche sur sulfate de magnésium, évapore. On recueille 20,8 g (100%) du produit attendu

### (c) acide 4-(1-adamantyl)-3-methoxybenzoïque.

Le composé précédent (11,3 g 35 mmoles) est dissous dans 200 ml de THF. La solution obtenue est ajoutée goutte à goutte sur du magnésium (1,3 g 52,5 mmoles) et un cristal d'iode. Après introduction, on chauffe à reflux pendant deux heures, refroidit à -78°C et fait passer un courant de CO₂ pendant une heure. On laisse remonter à température ambiante, verse le milieu réactionnel dans une solution aqueuse saturée en chlorure d'ammonium, extrait avec de l'éther éthylique, décante la phase organique, sèche sur sulfate de magnésium, évapore. Le résidu obtenu est purifié par chromatographie sur colonne de silice élué avec un mélange de dichlorométhane et d'éther éthylique (90-10). On recueille 5,1 g (51%) de l'acide attendu de point de fusion 259-60°C.

### (d) 4-(1-adamantyl)-3-methoxyacetophenone.

Dans un tricol et sous courant d'azote on introduit 2,8 g (10 mmoles) d'acide 4-(1-adamantyl)-3-méthoxybenzoïque et 100 ml d'éther éthylique anhydre. A -20°C, on ajoute goutte à goutte 15,3 ml (25 mmoles) de méthyllithium (1,6 M dans l'éther) puis agite pendant trois heures à température ambiante. On verse le milieu réactionnel dans une solution aqueuse saturée en chlorure d'ammonium, décante la phase organique, sèche sur sulfate de magnésium, évapore.On recueille 2,5 g (90%) de l'acetophenone attendu de point de fusion 89-90°C.

### (e) acide 4-[[3-oxo-3-[4-(1-adamantyl)-3-methoxyphényl]-1-propenyl]]benzoïque

Dans un ballon, on introduit 1,4 g (4,9 mmoles) de 4-(1-adamantyl)-3-methoxyacetophenone 810 mg (4,9 mmoles) d'acide 4-formylbenzoïque et 100 ml de méthanol. On ajoute 20 ml (20 mmoles) d'une solution de soude (1N) et agite à température ambiante pendant douze heures. On évapore à sec, reprend le milieu réactionnel par l'eau, acidifie à pH1, extrait avec de l'éther éthylique, décante la phase organique, sèche sur sulfate de magnésium, évapore. On triture le solide dans l'éther diisopropylique,filtre, sèche. On recueille 480 mg d'acide 4-[[3-oxo-3-[4-(1-adamantyl)-3-methoxyphényl]-1-propenyl]]benzoïque de point de fusion 277-9°C.

### EXEMPLE 12

### Acide 4-[4-(1-adamantyl)-3-methoxyethoxymethoxybenzoylthio]benzoïque.

### (a) 4-(1-adamantyl)-1-bromo-3-methoxyethoxymethoxybenzene.

De manière analogue à l'exemple 11(b) par réaction de 72,94 g (0,237 mole) de 2-(1-adamantyl)-5-bromophenol avec 32,5 ml (0,284 mole) de chlorure de méthoxyéthoxyméthyle, on obtient 86,9 g (93%) de 4-(1-adamantyl)-1-bromo-3-methoxyethoxymethoxybenzene de point de fusion 72-3°C.

### (b) acide 4-(1-adamantyl)-3-methoxyethoxymethoxybenzoïque.

Dans un tricol et sous courant d'azote, on introduit 43,5 g (0,11 mole) de 4-(1-adamantyl)-1-bromo-3-methoxyethoxymethoxybenzene et 450 ml de THF. A -78°C on ajoute goutte à goutte 48 ml (0,12 mole) d'une solution de n-butyillithium (2,5M dans l'hexane) et agite à cette température pendant une heure. A -70°C on introduit un courant de CO₂ pendant 30' et laisse revenir à température ambiante. On verse le milieu réactionnel dans une solution saturée de chlorure d'ammonium, extrait avec de l'acétate d'éthyle, décante la phase organique, sèche sur sulfate de magnésium, évapore. On triture le solide obtenu dans l'hexane, filtre, sèche. On recueille 27,8 g (82%) de l'acide attendu de point de fusion 145-7°C.

### (c) chlorure de 4-(1-adamantyl)-3-methoxyethoxymethoxybenzoyle.

Dans un ballon, on introduit une solution de 8 g (22 mmoles) d'acide 4-(1-adamantyl)-3-methoxyethoxymethoxybenzoïque dans 80 ml de dichlorométhane anhydre et ajoute 4,45 ml (22 mmoles) de dicyclohexylamine et agite pendant une heure. On ajoute ensuite 1,6 ml (22 mmoles) de chlorure de thionyle et agite une heure. On évapore à sec, reprend par l'éther éthylique anhydre, filtre le sel de dicyclohexylamine et évapore le filtrat. On recueille 8,5 g (100%) du chlorure d'acide brut qui sera utilisé tel quel pour la suite de la synthèse.

### (d) acide 4-[4-(1-adamantyl)-3-methoxyethoxymethoxybenzoylthio]benzoïque.

Dans un ballon, on introduit 850 mg (5,5 mmoles) d'acide 4-mercapto benzoïque et 20 ml de pyridine. On ajoute goutte à goutte une solution de 2,08 g (5,5 mmoles) de chlorure de 4-(1-adamantyl)-3-méthoxyéthoxyméthoxy benzoyle préparé précédemment et agite à température ambiante pendant six heures. On évapore à sec, reprend par eau et acétate d'éthyle,acidifie à pH 5, décante la phase organique, lave à l'eau, sèche sur sulfate de magnésium, évapore. Le solide obtenu est purifié par chromatographie sur colonne de silice élué avec un mélange de dichlorométhane et de méthanol (98-2). On recueille 1,5 g (57%) d'acide 4-[4-(1-adamantyl)-3-methoxyethoxymethoxybenzoylthio]benzoïque de point de fusion 219-21°C.

### EXEMPLE 13

### Acide 4-[4-(1-adamantyl)-3-methoxyethoxymethoxybenzoyloxy]benzoïque.

### (a) 4-[4-(1-adamantyl)-3-methoxyethoxymethoxybenzoyloxy]benzoate d'allyle.

Dans un tricol et sous courant d'azote, on introduit 1,26 g (5,5 mmoles) de 4-hydroxybenzoate de benzyle et 20 ml de THF. On ajoute par petites quantités 181 mg (6,1 mmoles) d'hydrure de sodium (80% dans l'huile) et agite jusqu'à cessation du dégagement gazeux. On ajoute ensuite goutte à goutte une solution de 2,08 g (5,5 mmoles) de chlorure de 4-(1-adamantyl)-3-méthoxyéthoxyméthoxybenzoyle préparé à l'exemple 12(c) et agite à température ambiante six heures. On verse le milieu réactionnel dans l'eau, extrait avec de l'éther éthylique, décante la phase organique, sèche sur sulfate de magnésium, évapore. On triture le solide obtenu dans un mélange d'hexane et d'éther éthylique, filtre, sèche. On recueille 2,47 g (79%) de l'ester benzylique attendu de point de fusion 107-8°C.

### (b) acide 4-[4-(1-adamantyl)-3-methoxyethoxymethoxybenzoyloxy]benzoïque.

Dans un réacteur, on introduit 2,46 g (4,3 mmoles) de 4-[4-(1-adamantyl)-3-methoxyethoxymethoxybenzoyloxy]benzoate d'allyle 40 ml de dioxanne et 492 mg de palladium sur charbon (10%). On hydrogène à 40°C et sous une pression de 7 bars pendant trois heures. On filtre le catalyseur, évapore à sec. On recristallise le solide obtenu dans le méthanol, filtre, sèche. On recueille 1,57 g (76%) de l'acide attendu de point de fusion 212-4°C.

### EXEMPLE 14

### Acide 4-[4-(1-adamantyl)-3-methoxyethoxymethoxybenzamido]benzoïque.

### (a) 4-[4-(1-adamantyl)-3-methoxyethoxymethoxybenzamido]benzoate d'allyle.

Dans un ballon, on introduit 1,95 g (11 mmoles) de 4-aminobenzoate d'allyle 1,7 ml (12 mmoles) de triéthylamine et 50 ml de THF. On ajoute goutte à goutte une solution de 4,16 g (11 mmoles) de chlorure de 4-(1-adamantyl)-3-méthoxyéthoxy méthoxybenzoyle et agite à température ambiante pendant 4 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec du dichlorométhane. On recueille 4,45 g (78%) de l'ester allylique attendu sous forme d'une huile.

### (b) acide 4-[4-(1-adamantyl)-3-methoxyethoxymethoxybenzamido]benzoïque.

Dans un ballon et sous courant d'azote, on introduit 107 mg (3,5 mmoles) d'hydrure de sodium (80% dans l'huile) et 5 ml de THF. On ajoute ensuite goutte à goutte 535 µl (3,5 mmoles) de malonate de diéthyle et agite jusqu'à cessation du dégement gazeux. Cette solution est introduite goutte à goutte dans un mélange de 1,67 g (3,2 mmoles) de 4-[4-(1-adamantyl)-3-methoxyethoxymethoxybenzamido] benzoate d'allyle 40 ml de THF et 184 mg ( 0,18 mmole) de tétrakis (triphénylphosphine)palladium(0) et agite à température ambiante pendant trois heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'acétate d'éthyle (80-20). Après évaporation des solvants, on recueille 630 mg (41%) d'acide 4-[4-(1-adamantyl)-3-methoxyethoxymethoxy benzamido]benzoïque de point de fusion 189-91°C.

### EXEMPLE 15

### Acide (E)-4-[2-(4-(1-adamantyl)-3-methoxyethoxymethoxyphenyl)ethenyl] benzoïque.

### (a) 4-(1-adamantyl)-3-methoxyethoxymethoxybenzaldéhyde.

Dans un tricol et sous courant d'azote, on introduit 43,1 g (0,109 mole) de 4-(1-adamantyl)-1-bromo-3-methoxyethoxymethoxybenzene et 450 ml de THF. A -78°C on ajoute goutte à goutte 48 ml (0,12 mole) d'une solution de n-butyillithium (2,5M dans l'hexane) et agite à cette température pendant une heure. A -70°C on introduit ensuite 9,25 ml (0,12 mole) de DMF et laisse revenir à température ambiante. On verse le milieu réactionnel dans une solution saturée de chlorure d'ammonium, extrait avec de l'acétate d'éthyle, décante la phase organique, sèche sur sulfate de magnésium, évapore. Le résidu obtenu est purifié par chromatographie sur colonne de silice élué avec du dichlorométhane. On recueille 27,8 g (74%) de l'aldéhyde attendu de point de fusion 87-9°C.

### (b) (E)-4-[2-(4-(1-adamantyl)-3-methoxyethoxymethoxyphenyl)ethenyl] benzoate de méthyle.

Dans un tricol et sous courant d'azote, on introduit 1,72 g (5 mmoles) de 4-(1-adamantyl)-3-methoxyethoxymethoxybenzaldéhyde 1,55 g (6 mmoles) de 4-méthoxycarbonylbenzylphosphonate de diméthyle 40 ml de THF et 224 mg d'éther couronne (15-crown-5). On ajoute ensuite par petites quantités 181 mg (6 mmoles) d'hydrure de sodium (80% dans l'huile) et agite à température ambiante pendant douze heures. On verse le milieu réactionnel dans l'eau, extrait avec de l'éther éthylique, décante la phase organique, sèche sur sulfate de magnésium, évapore.
Le résidu obtenu est purifié par chromatographie sur colonne de silice élué avec du dichlorométhane. On recueille 1,82 g (76%) d'ester méthylique attendu de point de fusion 113-4°C.

### (c) acide (E)4-[2-(4-(1-adamantyl)-3-methoxyethoxymethoxyphenyl)ethenyl] benzoïque.

Dans un ballon, on introduit 1,8 g (3,8 mmoles) de l'ester méthylique précédent 1,5 g (38 mmoles) d'hydroxyde de sodium et 50 ml de méthanol. On chauffe à reflux pendant trois heures, évapore à sec le milieu réactionnel. On reprend le résidu obtenu par l'eau, acidifie à pH 1, extrait avec de l'acétate d'éthyle, décante la phase organique, sèche sur sulfate de magnésium, évapore. On triture le solide dans le minimum d'éther éthylique, filtre, sèche. On recueille 1,63 g (93%) d'acide (E)-4-[2-(4-(1-adamantyl)-3-methoxyethoxymethoxyphenyl)ethenyl] benzoïque de point de fusion 219-21°C.

### EXEMPLE 16

### Acide (E)-4-[2-(4-(1-adamantyl)-3-methoxyethoxymethoxyphenyl)-1-propenyl] benzoïque.

### (a) 4-(1-adamantyl)-3-methoxyethoxymethoxyacétophenone.

Dans un tricol et sous courant d'azote on introduit 19,8 g (55 mmoles) d'acide 4-(1-adamantyl)-3-méthoxyéthoxyméthoxybenzoïque et 200 ml d'éther éthylique anhydre. A -20°C, on ajoute goutte à goutte 70 ml (0,13 mole)de méthyllithium (1,6 M dans l'éther) puis agite pendant trois heures à température ambiante. On verse le milieu réactionnel dans une solution aqueuse saturée en chlorure d'ammonium, décante la phase organique, sèche sur sulfate de magnésium, évapore.On recueille 19,7 g (100%) de l'acetophenone attendu sous forme d'une huile légèrement jaune.

### (b) (E)-4-[2-(4-(1-adamantyl)-3-methoxyethoxymethoxyphenyl)-1-propenyl]benzoate de méthyle.

De manière analogue à l'exemple 15(b) par réaction de 3,58 g (10 mmoles) de 4-(1-adamantyl)-3-methoxyethoxymethoxyacétophenone avec 3,13 g (12 mmoles) de 4-méthoxycarbonylbenzylphosphonate de diméthyle, on obtient après chromatographie sur colonne de silice élué avec un mélange d'heptane et d'acétate d'éthyle (80-20) 380 mg (15%) de (E)4-[2-(4-(1-adamantyl)-3-methoxyethoxy methoxyphenyl)-1-propenyl] benzoate de méthyle de point de fusion 84-6°C et 550 mg (22%) de (Z)-4-[2-(4-(1-adamantyl)-3-methoxyethoxymethoxyphenyl)-1-propenyl] benzoate de méthyle de point de fusion 77-8°C.

### (c) acide (E)-4-[2-(4-(1-adamantyl)-3-methoxyethoxymethoxyphenyl)-1-propenyl] benzoïque.

De manière analogue à l'exemple 15(c) à partir de 330 mg (0,67 mmole) de (E)-4-[2-(4-(1-adamantyl)-3-methoxyethoxy methoxyphenyl)-1-propenyl] benzoate de méthyle, on obtient 243 mg (76%) d'acide (E)-4-[2-(4-(1-adamantyl)-3-methoxy ethoxymethoxyphenyl)-1-propenyl] benzoïque de point de fusion 193-4°C.

### EXEMPLE 17

### Acide (Z)-4-[2-(4-(1-adamantyl)-3-methoxyethoxymethoxyphenyl)-1-propenyl] benzoïque.

De manière analogue à l'exemple 15(c) à partir de 540 mg (1,1 mmole) de (Z)-4-[2-(4-(1-adamantyl)-3-methoxyethoxy methoxyphenyl)-1-propenyl] benzoate de méthyle, on obtient 437 mg (83%) d'acide (Z)-4-[2-(4-(1-adamantyl)-3-methoxy ethoxymethoxyphenyl)-1-propenyl] benzoïque de point de fusion 191-2°C.

### EXEMPLE 18

### Acide 4-[4-(1-adamantyl)-3-methoxyethoxymethoxybenzoylmethyloxy] benzoïque.

### (a) 2'-bromo-4-(1-adamantyl)-3-hydroxyacétophenone.

Dans un ballon, on introduit 4,47 g (20 mmoles) de CuBr₂ et 45 ml de chloroforme. On chauffe à reflux et ajoute goutte à goutte une solution de 3,57 g (10 mmoles) de 4-(1-adamantyl)-3-methoxyethoxymethoxyacétophenone dans 50 ml d'acétate d'éthyle. On laisse le reflux pendant quatre heures, filtre le milieu réactionnel, évapore à sec. Le résidu est purifié par chromatographie sur colonne de silice élué avec du dichlorométhane. On recueille 2,51 g (72%) de 2'-bromo-4-(1-adamantyl)-3-hydroxyacétophenone de point de fusion 200-2°C.

### (b) 4-[4-(1-adamantyl)-3-hydroxybenzoylmethyloxy] benzoate de méthyle.

Dans un ballon, on introduit 2,15 g (6,1 mmoles) de 2'-bromo-4-(1-adamantyl)-3-hydroxyacétophenone 934 mg de 4-hydroxybenzoate de méthyle 1 g (7,37 mmoles) de carbonate de potassium et 60 ml de méthyléthylcétone. On chauffe à reflux pendant quatre heures, verse le milieu réactionnel dans l'acide chlorhydrique (1N), extrait avec de l'éther éthylique, décante la phase organique, sèche sur sulfate de magnésium, évapore. Le résidu obtenu est purifié par chromatographie sur colonne de silice élué avec un mélange d'heptane et d'acétate d'éthyle (70-30). On recueille 1,55 g (69%) du produit attendu de point de fusion 209-10°C.

### (c) 4-[4-(1-adamantyl)-3-methoxyethoxymethoxybenzoylmethyloxy] benzoate de méthyle.

De manière analogue à l'exemple 11(b) par réaction de 472 mg (1,12 mmole) de 4-[4-(1-adamantyl)-3-hydroxybenzoylmethyloxy] benzoate de méthyle avec 154 µl (1,35 mmole) de chlorure de méthoxyéthoxyméthyle, on obtient 217 mg (38%) de l'ester méthylique attendu sous forme d'une huile jaune.

### (d) acide 4-[4-(1-adamantyl)-3-methoxyethoxymethoxybenzoylmethyloxy] benzoïque.

De manière analogue à l'exemple 15 (c) à partir de 551 mg (1,08 mmole) de l'ester méthylique précédent, on obtient 230 mg (46%) d'acide 4-[4-(1-adamantyl)-3-methoxyethoxymethoxybenzoylmethyloxy]benzoïque de point de fusion 235-6°C.

### EXEMPLE 19

### Acide 4-[[3-oxo-3-[4-(1-adamantyl)-3-methoxyethoxymethoxyphényl]-1-propenyl]]benzoïque.

Dans un ballon, on introduit 1,8 g (5 mmoles) de 4-(1-adamantyl)-3-méthoxyéthoxyméthoxyacétophenone préparé à l'exemple 16(a) 820 mg (5 mmoles) de 4-formylbenzoate de méthyle et 20 ml de méthanol. On ajoute 10 mg de 18-crown-6 et 200 mg de soude en pastille et agite à température ambiante pendant 4 heures. On évapore à sec reprend par l'eau, acidifie à pH 3 avec de l'acide chlorhydrique (1 N), extrait avec de l'éther éthylique, décante la phase organique, sèche sur sulfate de magnésium, évapore. On purifie le résidu obtenu par chromatographie sur colonne de silice élué avec du dichlorométhane. On recueille 432 mg (17%) de 4-[[3-oxo-3-[4-(1-adamantyl)-3-methoxyethoxymethoxyphényl]-1-propenyl]]benzoate de méthyle de point de fusion 95-6°C et 636 mg (26%) d'acide 4-[[3-oxo-3-[4-(1-adamantyl)-3-methoxyethoxymethoxyphényl]-1-propenyl]]benzoïque de point de fusion 183-5°C.

### EXEMPLE 20

### Acide 4-[[3-hydroxy-3-[4-(1-adamantyl)-3-methoxyethoxymethoxyphényl]-1-propenyl]]benzoïque.

### (a) 4-[[3-hydroxy-3-[4-(1-adamantyl)-3-methoxyethoxymethoxyphényl]-1-propenyl]] benzoate de méthyle.

Dans un ballon, on introduit 425 mg (0,84 mmole) de 4-[[3-oxo-3-[4-(1-adamantyl)-3-methoxyethoxymethoxyphényl]-1-propenyl]]benzoate de méthyle obtenu à l'exemple 19, 246 mg de chlorure de cerium et 10 ml de méthanol. On agite à température ambiante pendant une heure puis ajoute 16 mg (0,42 mmole) de borohydrure de sodium et agite pendant quatre heures. On évapore à sec, reprend par eau, extrait avec de l'éther éthylique,décante la phase organique, sèche sur sulfate de magnésium, évapore. Le résidu obtenu est purifié par chromatographie sur colonne de silice élué avec du dichlorométhane. On recueille 344 mg (81%) de l'ester méthylique attendu sous forme d'une huile.

### (b) acide 4-[[3-hydroxy-3-[4-(1-adamantyl)-3-methoxyethoxymethoxyphényl]-1-propenyl]]benzoïque.

De manière analogue à l'exemple 15 (c) à partir de 308 mg (0,61 mmole) de l'ester méthylique précédent, on obtient 91 mg (30%) d'acide 4-[[3-hydroxy-3-[4-(1-adamantyl)-3-methoxyethoxymethoxyphényl]-1-propenyl]]benzoïque de point de fusion 127-9°C.

### EXEMPLE 21

### 4-[4-(1-adamantyl)-3-methoxyethoxymethoxythiobenzamido]benzoate d'allyle.

Dans un ballon, on introduit 2,9 g (5,6 mmoles) de 4-[4-(1-adamantyl)-3-methoxyethoxymethoxybenzamido]benzoate d'allyle 30 ml de toluène et ajoute 1,14 g (2,8 mmoles) de réactif de Lawesson. On chauffe à reflux pendant trois heures, évapore le milieu réactionnel à sec. On reprend le résidu obtenu par l'eau et le dichlorométhane, décante la phase organique, sèche sur sulfate de magnésium, évapore. Le résidu obtenu est purifié par chromatographie sur colonne de silice élué avec un mélange d'heptane et d'acétate d'éthyle (80-20). Après évaporation des solvants, on recueille 1 g (33%) de l'ester allylique attendu sous forme d'une huile orangée.

### EXEMPLE 22

Dans cet exemple, on a illustré diverses formulations concrètes à base des composés selon l'invention.

### A- VOIE ORALE

### (a) Comprimé de 0,2 g

- Composé de l'exemple 1 0,001 g
- Amidon 0,114 g
- Phosphate bicalcique 0,020 g
- Silice 0,020 g
- Lactose 0,030 g
- Talc 0,010 g
- Stéarate de magnésium 0,005 g

### (b) Suspension buvable en ampoules de 5 ml

- Composé de l'exemple 2 0,001 g
- Glycérine 0,500 g
- Sorbitol à 70% 0,500 g
- Saccharinate de sodium 0,010 g
- Parahydroxybenzoate de méthyle 0,040 g
- Arome qs
- Eau purifiée qsp 5 ml

### (c) Comprimé de 0,8 g

- Composé de l'exemple 6 0,500 g
- Amidon prégélatinisé 0,100 g
- Cellulose microcristalline 0,115 g
- Lactose 0,075 g
- Stéarate de magnésium 0,010 g

### (d) Suspension buvable en ampoules de 10 ml

- Composé de l'exemple 4 0,05 g
- Glycérine 1,000g
- Sorbitol à 70% 1,000g
- Saccharinate de sodium 0,010 g
- Parahydroxybenzoate de méthyle 0,080 g
- Arome qs
- Eau purifiée qsp 10 ml

### B- VOIE TOPIQUE

### (a) Onguent

- Composé de l'exemple 1 0,020 g
- Myristate d'isopropyle 81,700 g
- Huile de vaseline fluide 9,100 g
- Silice ("Aérosil 200" vendue par DEGUSSA) 9,180 g

### (b) Onguent

- Composé de l'exemple 6 0,300 g
- Vaseline blanche codex 100 g

### (c) Crème Eau-dans-Huile non ionique

- Composé de l'exemple 1 0,100 g
- Mélange d'alcools de lanoline émulsifs, de cires et d'huiles ("Eucerine anhydre" vendu par BDF) 39,900 g
- Parahydroxybenzoate de méthyle 0,075 g
- Parahydroxybenzoate de propyle 0,075 g
- Eau déminéralisée stérile qsp 100 g

### (d) Lotion

- Composé de l'exemple 1 0,100 g
- Polyéthylène glycol (PEG 400) 69,900 g
- Ethanol à 95% 30,000 g

### (e) Onguent hydrophobe

- Composé de l'exemple 2 0,300 g
- Mirystate d'isopropyle 36,400 g
- Huile de silicone ("Rhodorsil 47 V 300" vendu par RHONE-POULENC) 36,400 g
- Cire d'abeille 13,600 g
- Huile de silicone ("Abil 300.000 cst" vendu par GOLDSCHMIDT) 100g

### (f) Crème Huile-dans-Eau non ionique

- Composé de l'exemple 4 0,500 g
- Alcool cétylique 4,000 g
- Monostéarate de glycérole 2,500 g
- Stéarate de PEG 50 2,500 g
- Beurre de karité 9,200 g
- Propylène glycol 2,000 g
- Parahydroxybenzoate de méthyle 0,075 g
- Parahydroxybenzoate de propyle 0,075 g
- Eau déminéralisée stérile 100 g

## Revendications

1. Composés bicycliques aromatiques, caractérisés par le fait qu'ils répondent à la formule générale (I) suivante : 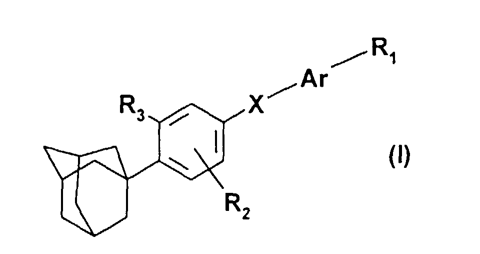 dans laquelle:
- R₁ représente
(i) le radical -CH₃
(ii) le radical -(CH₂)n-O-R₄
(iii) le radical -O-(CH₂)m-(CO)n-R₅
(iv) le radical -CO-R₆
(v) le radical -CO-O-R₇
les valeurs m et n, ainsi que les différents radicaux R₄ à R₇ ayant la signification donnée ci-après,
- R₂ représente un atome d'hydrogène, d'halogène, un radical alkyle linéaire ou ramifié ayant de 1 à 20 atomes de carbone, un radical -OR₄, un radical -O-CH₂-O-CH₂-CH₂-O-CH₃.
- R₃ représente:
(i) un radical -Y-(CH₂)p-Y-(CH₂)q-R₈
(ii) un radical -(CH₂)p-(Y)s-(CH₂)q-R₈
(iii) un radical -Y-(CH₂)q-R₈
(v) un radical -CH=CH-(CH₂)r-R₈
les valeurs p, q, r, s et les radicaux Y et R₈ ayant la signification donnée ci-après,
- X représente les liaisons de formules (a)-(m) suivantes pouvant être lues dans les deux sens : 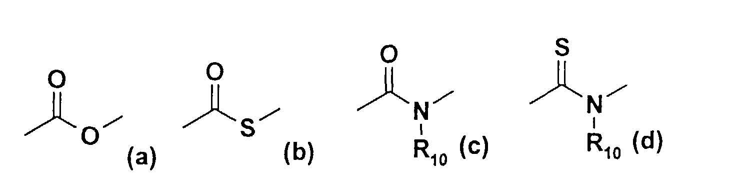 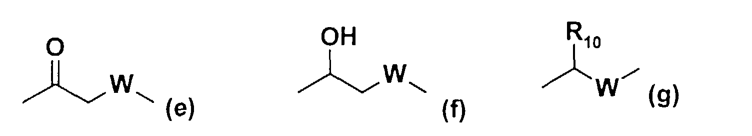 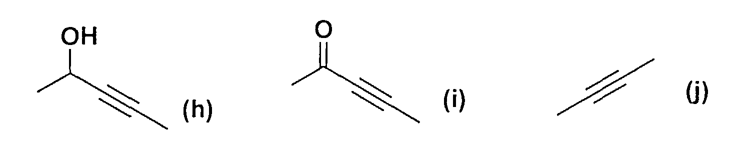 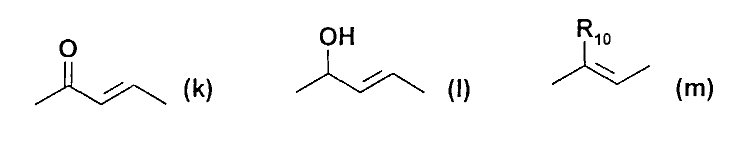
- Ar représente un radical choisi parmi les radicaux de formules (a)-(f) suivantes : 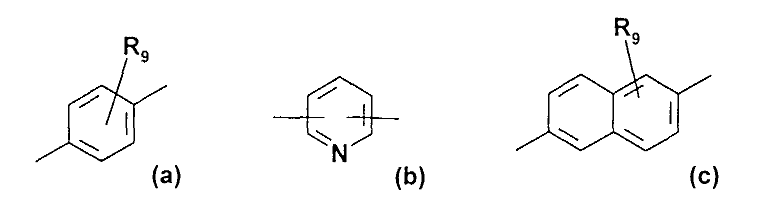 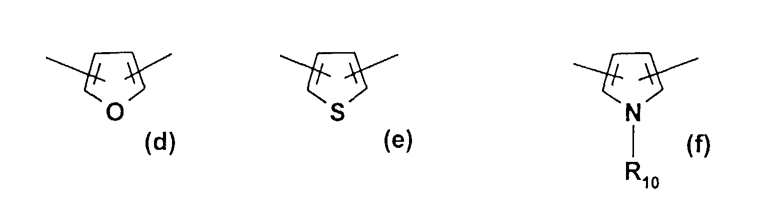 étant entendu que dans tout ce qui précède :
- m est un nombre entier égal à 1,2 ou 3
- n est un nombre entier égal à 0 ou 1
- p est un nombre entier compris inclusivement entre 1 et 12
- q est un nombre entier compris inclusivement entre 0 et 12
- r est un nombre entier compris inclusivement entre 0 et 10.
- s est un nombre entier égal à 0 ou 1
- t est un nombre entier égal à 0, 1 ou 2
- Y représente l'atome d'oxygène ou le radical S(O)t
- W représente l'atome d'oxygène, le radical S(O)t ou le radical N-R₁₀
- R₄ représente un atome d'hydrogène, un radical alkyle inférieur ou un radical -CO-R₁₁,
- R₅ représente un alkyle inférieur ou un hétérocycle,
- R₆ représente un atome d'hydrogène, un radical alkyle inférieur ou un radical 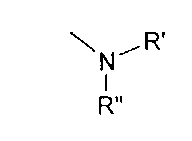 dans lequel R' et R" représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- R₇ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- R8 représente un atome d'hydrogène, un radical alkyle ramifié ayant de 1 à 20 atomes de carbone, un radical cycloaliphatique en C3-C6, un radical monohydroxyalkyle ou un radical polyhydroxyalkyle dont les hydroxy sont éventuellement protégés sous forme de méthoxy, d'acétoxy ou d'acétonide, un radical aryle, un radical alkényle, un radical alkynyle, un radical -CO-R₆, un radical -CO-O-R₇, un radical aminoalkyle dont la fonction amine est éventuellement substituée par un ou deux groupements alkyles inférieurs, un hétérocycle,
- R₉ représente un atome d'hydrogène,d'halogène, un radical allkyle linéaire ou ramifié ayant de 1 à 20 atomes de carbone, un radical -OR₄, un radical -O-CH₂-O-CH₂-CH₂-O-CH₃,
- R₁₀, identique ou différent, représente un atome d'hydrogène, un radical alkyle inférieur,
- R₁₁ représente un radical alkyle inférieur,
étant donné que lorsque X représente la liaison de formule (e) et R₃ représente le radical (iii) dans lequel Y représente un atome d'oxygène et R₈ représente un atome d'hydrogène, alors q doit être supérieur à 6,
ainsi que leurs sels et leurs isomères optiques et géométriques.

2. Composés selon la revendication 1, caractérisés par le fait qu'ils se présentents sous forme de sels d'un métal alcalin ou alcalino-terreux, ou encore de zinc ou d'une amine organique.

3. Composés selon l'une des revendications 1 ou 2, caractérisés par le fait que les radicaux alkyles inférieurs sont choisis parmi le groupe constitué par les radicaux méthyle, éthyle, isopropyle, butyle, tertiobutyle, hexyle, nonyle et dodécyle .

4. Composés selon l'une des revendications précédentes, caractérisés en ce que les radicaux alkyles linéaires ou ramifiés ayant de 1 à 20 atomes de carbone sont choisis parmi le groupe constitué par les radicaux méthyle, éthyle, propyle, 2-éthyl-hexyle, octyle, dodécyle, hexadécyle et octadécyle.

5. Composés selon l'une des revendications précédentes, caractérisés en ce que les radicaux monohydroxyalkyles sont choisis parmi le groupe constitué par les radicaux 2-hydroxyéthyle, 2-hydroxypropyle ou 3-hydroxypropyle.

6. Composés selon l'une des revendications précédentes, caractérisés en ce que les radicaux polyhydroxyalkyles sont choisis parmi le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une des revendications précédentes, caractérisés en ce que le radical aryle est un radical phényle éventuellement substitué par au moins un atome d'halogène, un hydroxyle ou une fonction nitro.

8. Composés selon l'une des revendications précédentes, caractérisés en ce que les radicaux aralkyles sont choisis parmi le groupe constitué par les radicaux benzyle ou phénéthyle, éventuellement substitués par au moins un atome d'halogène, un hydroxyle ou une fonction nitro.

9. Composés selon l'une des revendications précédentes, caractérisés en ce que les radicaux alkényles sont choisis dans le groupe constitué par les radicaux contenant de 1 à 5 atomes de carbone et présentant une ou plusieurs insaturations éthyléniques, et en particulier le radical allyle.

10. Composés selon l'une des revendications précédentes, caractérisés en ce que les restes de sucre sont choisis dans le groupe constitué par les restes de glucose, de galactose, de mannose et d'acide glucuronique.

11. Composés selon l'une quelconque des revendications précédentes, caractérisés en ce que les restes d'aminoacide sont choisis dans le groupe constitué par les restes dérivant de la lysine, de la glycine ou de l'acide aspartique.

12. Composés selon l'une quelconque des revendications précédentes, caractérisés en ce que les restes de peptides sont choisis dans le groupe constitué par les restes de dipeptides ou de tripeptides.

13. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

14. Composés selon l'une quelconque des revendications précédentes, caractérisés en ce que les atomes d'halogène sont choisis dans le groupe constitué par le fluor, le chlore et le brome.

15. Composés selon l'une quelconque des revendications précédentes, caractérisés en ce que le radical alkyle ramifié ayant de 1 à 20 atomes de carbone est choisi parmi les radicaux 2-méthylbutyle, 2-méthylpentyle, 1-méthylhexyle, 3-méthylheptyle.

16. Composés selon l'une quelconque des revendications précédentes, caractérisés en ce que le radical aminoalkyle est choisi parmi les radicaux aminométhyle, 3-aminopropyle, 6-aminohexyle.

17. Composés selon l'une quelconque des revendications précédentes, caractérisés en ce que le radical alkynyle présente de 2 à 6 atomes de carbone.

18. Composés selon l'une quelconque des revendications précédentes, caractérisés en ce que les radicaux cycloaliphatiques de 3 à 6 atomes de carbone sont choisis parmi le radical cyclopropyle et le radical cyclohexyle.

19. Composés selon la revendication 1, caractérisés par le fait qu'ils sont pris, seuls ou en mélanges, dans le groupe constitué par :
Acide 4-[-4-(1-adamantyl)-3-méthoxybenzoyloxy]benzoïque.
Acide (E)-4-[[2-[4-(1-adamantyl)-3-hydroxyphényl]éthényl]]benzoïque.
Acide (E)-4-[[2-[4-(1-adamantyl)-3-propyloxyphényl]éthényl]]benzoïque.
Acide (E)-4-[[2-[4-(1-adamantyl)-3-heptyloxyphényl]éthényl]]benzoïque.
Acide (E)-4-[[2-[4-(1-adamantyl)-3-méthoxyphényl]éthènyl]]benzoïque.
Acide 4-[4-(1-adamantyl)-3-méthoxyéthoxyméthoxyphénylèthynyl]benzoïque.
Acide (E)-4-[[2-[4-(1-adamantyl)-3-(5-carbamoylpentyloxy)phényl]éthényl]] benzoïque.
Acide (E)-4-[[2-[4-(1-adamantyl)-3-méthoxyphényl]1-propényl]]benzoïque.
Acide (E)-4-[[2-[4-(1-adamantyl)-3-(3-hydroxypropyloxy)phényl]éthényl]] benzoïque.
Acide (E)-4-[[2-[4-(1-adamantyl)-3-(6-hydroxyhexyloxy)phényl]éthényl]] benzoïque.
Acide 4-[[3-oxo-3-[4-(1-adamantyl)-3-methoxyphényl]-1-propenyl]]benzoïque.
Acide 4-[4-(1-adamantyl)-3-methoxyethoxymethoxybenzoylthio]benzoïque.
Acide 4-[4-(1-adamantyl)-3-methoxyethoxymethoxybenzoyloxy]benzoïque.
Acide 4-[4-(1-adamantyl)-3-methoxyethoxymethoxybenzamido]benzoïque.
Acide (E)4-[2-(4-(1-adamantyl)-3-methoxyethoxymethoxyphenyl)ethenyl] benzoïque.
Acide (E)-4-[2-(4-(1-adamantyl)-3-methoxyethoxymethoxyphenyl)-1-propenyl] benzoïque.
Acide (Z)-4-[2-(4-(1-adamantyl)-3-methoxyethoxymethoxyphenyl)-1-propenyl] benzoïque.
Acide 4-[4-(1-adamantyl)-3-methoxyethoxymethoxybenzoylmethyloxy]benzoïque.
Acide 4-[[3-oxo-3-[4-(1-adamantyl)-3-methoxyethoxymethoxyphényl]-1-propynyl]]benzoïque.
Acide 4-[[3-hydroxy-3-[4-(1-adamantyl)-3-methoxyethoxymethoxyphényl]-1-propynyl]]benzoïque.
Acide (E)-4-[[3-oxo-3-[4-(1-adamantyl)-3-methoxyethoxymethoxyphenyl]-1-propenyl]]benzoïque.
Acide 4-[[3-hydroxy-3-[4-(1-adamantyl)-3-methoxyethoxymethoxyphenyl]-1-propenyl]]benzoïque.
4-[4-(1-adamantyl)-3-methoxyethoxymethoxythiobenzamido]benzoate d'allyle.
Acide 4-[[3-hydroxy-3-[4-(1-adamantyl)-3-methoxyphenyl]-1-propynyl]]benzoïque.
Acide 2-hydroxy-4-[[3-hydroxy-3-[4-(1-adamantyl)-3-methoxyphenyl]-1-propynyl]]benzoïque.
Acide 2-hydroxy-4-[[3-hydroxy-3-[4-(1-adamantyl)-3-methoxyethoxymethoxy phenyl]-1-propynyl]]benzoïque.
4-[[3-hyd roxy-3-[4-(1-adamantyl)-3-methoxyethoxymethoxyphenyl]1-propynyl]]benzaldehyde.
4-[[3-hydroxy-3-[4-(1-adamantyl)-3-methoxyethoxymethoxyphenyl]-1-propynyl]]benzenemethanol.
N-ethyl-4-[4-(1-adamantyl)-3-méthoxyéthoxyméthoxyphényléthynyl]benzamide.
N-(4-hydroxyphenyl)-4-[4-(1-adamantyl)-3-méthoxyéthoxyméthoxyphényl éthynyl]benzamide.
4-[4-(1-adamantyl)-3-méthoxyéthoxyméthoxyphényléthynyl]phenol.

20. Composés selon la revendication 1, caractérisés par le fait qu'ils présentent l'une au moins des caractéristiques suivantes :
- R₁ est le radical
-COO-R₇ et -CO-R₆.
- R₃ est le radical
-Y-(CH₂)p-Y-(CH₂)q-R₈.
-(CH₂)p-(Y)n-(CH₂)q-R₈.
-Y-(CH₂)q-R₈.
- X représente la liaison de formule (a), (h), (i), (j), (k) ou (m).
- Ar représente le radical de formule (a) ou (b).

21. Composés selon l'une quelconque des revendications précédentes pour une utilisation comme médicament.

22. Composés selon la revendication 21 pour une utilisation comme médicament destiné au traitement des affections dermatologiques, rhumatismales, respiratoires, cardiovasculaires et ophtalmologiques.

23. Utilisation de l'un au moins des composés définis aux revendications 1 à 20 pour la fabrication d'un médicament destiné au traitement des affections dermatologiques, rhumatismales, respiratoires, cardiovasculaires et ophtalmologiques.

24. Composition pharmaceutique, caractérisée par le fait qu'elle comprend, dans un support pharmaceutiquement acceptable, au moins l'un des composés tels que définis à l'une quelconque des revendications 1 à 20.

25. Composition selon la revendication 24, caractérisée en ce que la concentration en composé(s) selon l'une des revendication 1 à 16 est comprise entre 0,001 % et 5 % en poids par rapport à l'ensemble de la composition.

26. Composition cosmétique, caractérisée par le fait qu'elle comprend, dans un support cosmétiquement acceptable, au moins l'un des composés tels que définis à l'une quelconque des revendications 1 à 20.

27. Composition selon la revendication 26, caractérisée en ce que la concentration en composé(s) selon l'une des revendications 1 à 20 est comprise entre 0,001 % et 3 % en poids par rapport à l'ensemble de la composition.

28. Utilisation d'une composition cosmétique telle que définie à l'une des revendications 26 ou 27 pour l'hygiène corporelle ou capillaire.

## Patentansprüche

1. Aromatische bicyclische Verbindungen,
dadurch gekennzeichnet, daß
sie der folgenden allgemeinen Formel (I) entsprechen: 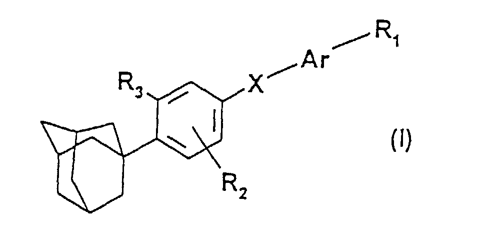 worin bedeuten:
- R₁:
(i) die Gruppe -CH₃
(ii) eine Gruppe -(CH₂)ₙ-O-R₄
(iii) eine Gruppe -O-(CH₂)ₘ-(CO)ₙ-R₅
(iv) eine Gruppe -CO-R₆
(v) eine Gruppe -CO-O-R₇,
wobei die Werte m und n sowie die verschiedenen Gruppen R₄ bis R₇ die nachstehend angegebenen Bedeutungen aufweisen,
- R₂: ein Wasserstoffatom, ein Halogenatom, eine geradkettige oder verzweigte Alkylgruppe mit 1 bis 20 Kohlenstoffatomen, eine Gruppe -OR₄, eine Gruppe -O-CH₂-O-CH₂-CH₂-O-CH₃,
- R₃:
(i) eine Gruppe -Y-(CH₂)ₚ-Y-(CH₂)_{q}-R₈
(ii) eine Gruppe -(CH₂)ₚ-(Y)ₛ-(CH₂)_{q}-R₈
(iii) eine Gruppe -Y-(CH₂)_{q}-R₈
(iv) eine Gruppe -CH=CH-(CH₂)ᵣ-R₈
wobei die Werte p, q, r und s und die Gruppen Y und R₈ die nachfolgend angegebenen Bedeutungen aufweisen,
- X die Bindungen der folgenden Formeln (a) - (m), die in beiden Richtungen gelesen werden können: 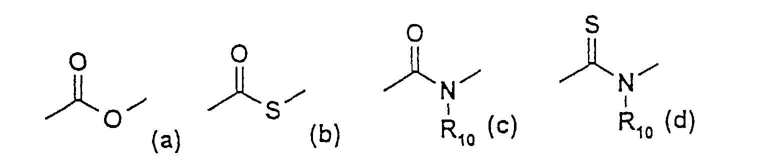 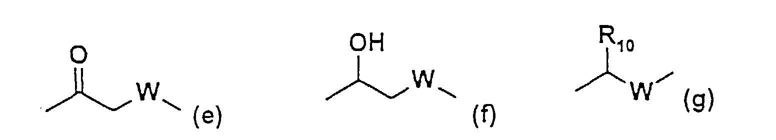 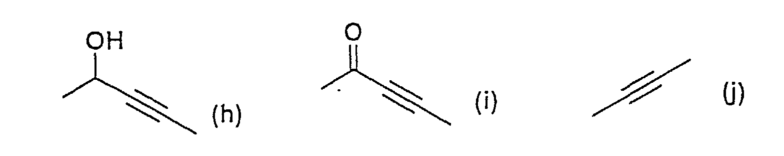 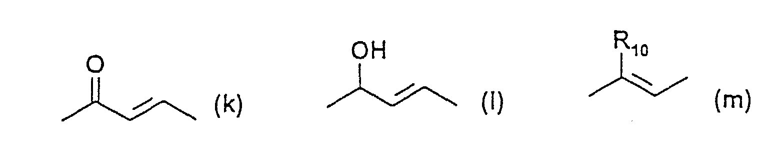
- Ar eine Gruppe, die unter den folgenden Gruppen der Formeln (a) - (f) ausgewählt ist: 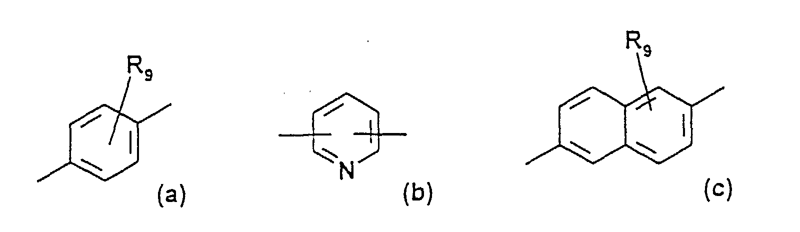 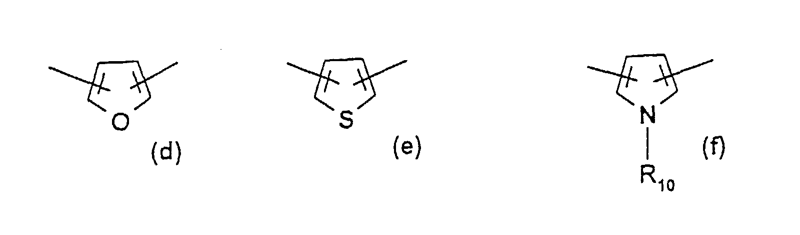 mit der Maßgabe, daß:
- m eine ganze Zahl 1, 2 oder 3 ist,
- n 0 oder 1 bedeutet,
- p eine ganze Zahl im Bereich von 1 bis 12 ist,
- q 0 oder eine ganze Zahl im Bereich von 1 bis 12 bedeutet,
- r 0 oder eine ganze Zahl im Bereich von 1 bis 10 bedeutet,
- s 0 oder 1 ist,
- t 0, 1 oder 2 ist,
- Y Sauerstoff oder die Gruppe S(O)ₜ bedeutet,
- W Sauerstoff, die Gruppe S(O)ₜ oder die Gruppe N-R₁₀ bedeutet,
- R₄ Wasserstoff, eine niedere Alkylgruppe oder eine Gruppe -CO-R₁₁ bedeutet,
- R₅ eine niedere Alkylgruppe oder einen Heterocyclus bedeutet,
- R₆ Wasserstoff, eine niedere Alkylgruppe oder eine Gruppe 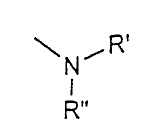 bedeutet, wobei R' und R" ein Wasserstoffatom, eine niedere Alkylgruppe, eine Mono- oder Polyhydroxyalkylgruppe, eine Arylgruppe, die gegebenenfalls substituiert ist, oder einen Aminosäurerest, Peptidrest oder Zuckerrest bedeuten oder gemeinsam einen Heterocyclus bilden,
- R₇ Wasserstoff, eine geradkettige oder verzweigte Alkylgruppe mit 1 bis 20 Kohlenstoffatomen, eine Alkenylgruppe, eine Mono- oder Polyhydroxyalkylgruppe, eine gegebenenfalls substituierte Aryl- oder Aralkylgruppe oder einen Zucker-, Aminosäure- oder Peptidrest bedeutet,
- R₈ ein Wasserstoffatom, eine verzweigte Alkylgruppe mit 1 bis 20 Kohlenstoffatomen, eine cycloaliphatische Gruppe mit 3 bis 6 Kohlenstoffatomen, eine Monohydroxyalkylgruppe oder eine Polyhydroxyalkylgruppe, deren Hydroxygruppen gegebenenfalls in Form von Methoxy, Acetoxy oder Acetonid geschützt sind, eine Arylgruppe, eine Alkenylgruppe, eine Alkinylgruppe, eine Gruppe -CO-R₆, eine Gruppe -CO-O-R₇, eine Aminoalkylgruppe, deren Aminogruppe gegebenenfalls mit einer oder zwei niederen Alkylgruppen substituiert ist, oder einen Heterocyclus bedeutet,
- R₉ ein Wasserstoffatom, ein Halogenatom, eine geradkettige oder verzweigte Alkylgruppe mit 1 bis 20 Kohlenstoffatomen, eine Gruppe -OR₄, eine Gruppe-O-CH₂-O-CH₂-, CH₂-O-CH₃ bedeutet,
- die Gruppen R₁₀, die identisch oder voneinander verschieden sind, Wasserstoff oder eine niedere Alkylgruppe bedeuten,
- R₁₁ eine niedere Alkylgruppe bedeutet,
mit der Maßgabe, daß:
q über 6 liegen muß, wenn X die Bindung der Formel (e) bedeutet, R₃ die Gruppe (iii) bedeutet, worin Y ein Sauerstoffatom ist, und R₈ Wasserstoff bedeutet;
sowie die Salze und optischen und geometrischen Isomere dieser Verbindungen.

2. Verbindungen nach Anspruch 1, dadurch gekennzeichnet, daß sie in Form eines Alkali- oder Erdalkalimetallsalzes oder auch in Form eines Zinksalzes oder Salzes eines organischen Amins vorliegen.

3. Verbindungen nach einem der Ansprüche 1 oder 2, dadurch gekennzeichnet, daß die niederen Alkylgruppen unter Methyl, Ethyl, Isopropyl, Butyl, tert.-Butyl, Hexyl, Nonyl und Dodecyl ausgewählt sind.

4. Verbindungen nach einem der vorhergehenden Ansprüche, dadurch gekennzeichnet, daß die geradkettigen oder verzweigten Alkylgruppen mit 1 bis 20 Kohlenstoffatomen unter den Gruppen Methyl, Ethyl, Propyl, 2-Ethylhexyl, Octyl, Dodecyl, Hexadecyl und Octadecyl ausgewählt sind.

5. Verbindungen nach einem der vorhergehenden Ansprüche, dadurch gekennzeichnet, daß die Monohydroxyalkylgruppen unter den Gruppen 2-Hydroxyethyl, 2-Hydroxypropyl oder 3-Hydroxypropyl ausgewählt sind.

6. Verbindungen nach einem der vorhergehenden Ansprüche, dadurch gekennzeichnet, daß die Polyhydroxyalkylgruppen unter den Gruppen 2,3-Dihydroxypropyl, 2,3,4-Trihydroxybutyl, 2,3,4,5-Tetrahydroxypentyl oder Pentaerythrit ausgewählt sind.

7. Verbindungen nach einem der vorhergehenden Ansprüche, dadurch gekennzeichnet, daß die Arylgruppe eine Phenylgruppe ist, die gegebenenfalls mit mindestens einem Halogenatom, einer Hydroxygruppe oder einer Nitrogruppe substituiert ist.

8. Verbindungen nach einem der vorhergehenden Ansprüche, dadurch gekennzeichnet, daß die Aralkylgruppen unter den Gruppen Benzyl oder Phenethyl ausgewählt sind, die gegebenenfalls mit mindestens einem Halogenatom, einer Hydroxygruppe oder einer Nitrogruppe substituiert sind.

9. Verbindungen nach einem der vorhergehenden Ansprüche, dadurch gekennzeichnet, daß die Alkenylgruppen unter den Gruppen mit 1 bis 5 Kohlenstoffatomen, die eine oder mehrere ethylenisch ungesättigte Bindungen aufweisen, und insbesondere der Allylgruppe ausgewählt sind.

10. Verbindungen nach einem der vorhergehenden Ansprüche, dadurch gekennzeichnet, daß die Zuckerreste unter den Resten von Glucose, Galactose, Mannose und Glucuronsäure ausgewählt sind.

11. Verbindungen nach einem der vorhergehenden Ansprüche, dadurch gekennzeichnet, daß die Aminosäurereste unter den Resten ausgewählt sind, die von Lysin, Glycin oder Asparaginsäure abgeleitet sind.

12. Verbindungen nach einem der vorhergehenden Ansprüche, dadurch gekennzeichnet, daß die Peptidreste unter den Resten von Dipeptiden oder Tripeptiden ausgewählt sind.

13. Verbindungen nach einem der vorhergehenden Ansprüche, dadurch gekennzeichnet, daß die heterocyclischen Gruppen unter den Gruppen Piperidino, Morpholino, Pyrrolidino oder Piperazino, die gegebenenfalls in 4-Stellung mit einer C₁₋₆-Alkylgruppe oder Mono- oder Polyhydroxyalkylgruppe substituiert sind, ausgewählt sind.

14. Verbindungen nach einem der vorhergehenden Ansprüche, dadurch gekennzeichnet, daß die Halogenatome unter Fluor, Chlor und Brom ausgewählt sind.

15. Verbindungen nach einem der vorhergehenden Ansprüche, dadurch gekennzeichnet, daß die verzweigte Alkylgruppe mit 1 bis 20 Kohlenstoffatomen unter den Gruppen 2-Methylbutyl, 2-Methylpentyl, 1-Methylhexyl und 3-Methylheptyl ausgewählt ist.

16. Verbindungen nach einem der vorhergehenden Ansprüche, dadurch gekennzeichnet, daß die Aminoalkylgruppe unter den Gruppen Aminomethyl, 3-Aminopropyl und 6-Aminohexyl ausgewählt ist.

17. Verbindungen nach einem der vorhergehenden Ansprüche, dadurch gekennzeichnet, daß die Alkenylgruppe 2 bis 6 Kohlenstoffatome aufweist.

18. Verbindungen nach einem der vorhergehenden Ansprüche, dadurch gekennzeichnet, daß die cycloaliphatischen Gruppen mit 3 bis 6 Kohlenstoffatomen unter den Gruppen Cyclopropyl und Cyclohexyl ausgewählt sind.

19. Verbindungen nach Anspruch 1, dadurch gekennzeichnet, daß sie alleine oder im Gemisch ausgewählt sind unter:
4-[4-(1-Adamantyl)-3-methoxybenzoyloxy]-benzoesäure
(E)-4-[[2-[4-(1-Adamantyl)-3-hydroxyphenyl]-ethenyl]]-benzoesäure
(E)-4-[[2-[4-(1-Adamantyl)-3-propyloxyphenyl]-ethenyl]]-benzoesäure
(E)-4-[[2-[4-(1-Adamantyl)-3-heptyloxyphenyl]-ethenyl]]-benzoesäure
(E)-4-[[2-[4-(1-Adamantyl)-3-methoxyphenyl]-ethenyl]]-benzoesäure
4-[4-(1-Adamantyl)-3-methoxyethoxymethoxyphenylethinyl]-benzoesäure
(E)-4-[[2-[4-(1-Adamantyl)-3-(5-carbamoylpentyloxy)-phenyl]-ethenyl]]-benzoesäure
(E)-4-[[2-[4-(1-Adamantyl)-3-methoxyphenyl]-1-propenyl]]-benzoesäure
(E)-4-[[2-[4-(1-Adamantyl)-3-(3-hydroxypropyloxy)-phenyl]-ethenyl]]-benzoesäure
(E)-4-[[2-[4-(1-Adamantyl)-3-(6-hydroxyhexyloxy)phenyl]-ethenyl]]-benzoesäure
4-[[3-Oxo-3-[4-(1-adamantyl)-3-methoxyphenyl]-1-propenyl]]-benzoesäure
4-[4-(1-Adamantyl)-3-methoxyethoxymethoxybenzoylthio]-benzoesäure
4-[4-(1-Adamantyl)-3-methoxyethoxymethoxybenzoyloxy]-benzoesäure
4-[4-(1-Adamantyl)-3-methoxyethoxymethoxybenzamido]-benzoesäure
(E)-4-[2-(4-(1-Adamantyl)-3-methoxyethoxymethoxyphenyl)-ethenyl]-benzoesäure
(E)-4-[2-(4-(1-Adamantyl)-3-methoxyethoxymethoxyphenyl)-1-propenyl]-benzoesäure
(Z)-4-[2-(4-(1-Adamantyl)-3-methoxyethoxymethoxyphenyl)-1-propenyl]-benzoesäure
4-[4-(1-Adamantyl)-3-methoxyethoxymethoxybenzoylmethyloxy]-benzoesäure
4-[[3-Oxo-3-[4-(1-adamantyl)-3-methoxyethoxymethoxyphenyl]-1-propinyl]]-benzoesäure
4-[[3-Hydroxy-3-[4-(1-adamantyl)-3-methoxyethoxymethoxyphenyl]-1-propinyl]]-benzoesäure
(E)-4-[[3-Oxo-3-[4-(1-adamantyl)-3-methoxyethoxymethoxyphenyl]-1-propenyl]]-benzoesäure
4-[[3-Hydroxy-3-[4-(1-adamantyl)-3-methoxyethoxymethoxyphenyl]-1-propenyl]]-benzoesäure
Allyl-4-[4-(1-adamantyl)-3-methoxyethoxymethoxythiobenzamido]-benzoat
4-[[3-Hydroxy-3-[4-(1-adamantyl)-3-methoxyphenyl]-1-propinyl]]-benzoesäure
2-Hydroxy-4-[[3-hydroxy-3-[4-(1-adamantyl)-3-methoxyphenyl]-1-propinyl]]-benzoesäure
2-Hydroxy-4-[[3-hydroxy-3-[4-(1-adamantyl)-3-methoxyethoxymethoxyphenyl]-1-propinyl]]-benzoesäure
4-[[3-Hydroxy-3-[4-(1-adamantyl)-3-methoxyethoxymethoxyphenyl]-1-propinyl]]-benzaldehyd
4-[[3-Hydroxy-3-[4-(1-adamantyl)-3-methoxyethoxymethoxyphenyl]-1-propinyl]]-benzolmethanol
N-Ethyl-4-[4-(1-adamantyl)-3-methoxyethoxymethoxyphenylethinyl]-benzamid
N-(4-Hydroxyphenyl)-4-[4-(1-adamantyl)-3-methoxyethoxymethoxyphenylethinyl]-benzamid
4-[4-(1-Adamantyl)-3-methoxyethoxymethoxyphenylethinyl]-phenol

20. Verbindungen nach Anspruch 1, dadurch gekennzeichnet, daß sie mindestens eine der folgenden Eigenschaften aufweisen:
- R₁ ist die Gruppe
-COO-R₇ und -CO-R₆.
- R₃ ist die Gruppe
-Y-(CH₂)ₚ-Y-(CH₂)_{q}-R₈.
-(CH₂)ₚ-(Y)ₙ-(CH₂)_{q}-R₈.
-Y-(CH₂)_{q}-R₈.
- X ist eine Bindung der Formel (a), (h), (i), (j), (k) oder (m).
- Ar bedeutet die Gruppe der Formel (a) oder (b).

21. Verbindungen nach einem der vorhergehenden Ansprüche zur Verwendung als Arzneimittel.

22. Verbindungen nach Anspruch 21 zur Verwendung als Arzneimittel, das zur Behandlung von dermatologischen Erkrankungen, rheumatischen Erkrankungen, Erkrankungen der Atemwege, kardiovaskulären Erkrankungen und ophtalmologischen Erkrankungen bestimmt ist.

23. Verwendung mindestens einer Verbindung nach einem der Ansprüche 1 bis 20 zur Herstellung eines Arzneimittels, das zur Behandlung von dermatologischen Erkrankungen, rheumatischen Erkrankungen, Erkrankungen der Atemwege, kardiovaskulären Erkrankungen und ophtalmologischen Erkrankungen bestimmt ist.

24. Pharmazeutische Zusammensetzung, dadurch gekennzeichnet, daß sie in einem pharmazeutisch akzeptablen Träger mindestens eine Verbindung nach einem der Ansprüche 1 bis 20 enthält.

25. Zusammensetzung nach Anspruch 24, dadurch gekennzeichnet, daß die Konzentration der Verbindung(en) nach einem der Ansprüche 1 bis 16 im Bereich von 0,001 bis 5 Gew.-%, bezogen auf die gesamte Zusammensetzung, liegt.

26. Kosmetische Zusammensetzung, dadurch gekennzeichnet, daß sie in einem kosmetisch akzeptablen Träger mindestens eine Verbindung nach einem der Ansprüche 1 bis 20 enthält.

27. Zusammensetzung nach Anspruch 26, dadurch gekennzeichnet, daß die Konzentration der Verbindung(en) nach einem der Ansprüche 1 bis 20 im Bereich von 0,001 bis 3 Gew.-%, bezogen auf die gesamte Zusammensetzung, liegt.

28. Verwendung einer kosmetischen Zusammensetzung nach einem der Ansprüche 26 oder 27 zur Körper- oder Haarhygiene.

## Claims

1. Bicyclic aromatic compounds, characterized in that they correspond to the general formula (I) below: 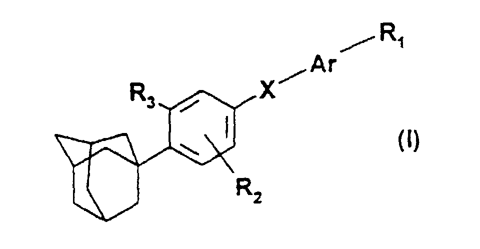 in which:
- R₁ represents
(i) the -CH₃ radical
(ii) the radical -(CH₂)n-O-R₄
(iii) the radical -O-(CH₂)m-(CO)n-R₅
(iv) the radical -CO-R₆
(v) the radical -CO-O-R₇
the values m and n, as well as the various radicals R₄ to R₇, having the meaning given below,
- R₂ represents a hydrogen or halogen atom, a linear or branched alkyl radical having from 1 to 20 carbon atoms, a radical -OR₄ or an -O-CH₂-O-CH₂-CH₂-O-CH₃ radical,
- R₃ represents:
(i) a radical -Y-(CH₂)p-Y-(CH₂)q-R₈
(ii) a radical -(CH₂)p-(Y)s-(CH₂)q-R₈
(iii) a radical -Y-(CH₂)q-R₈
(v) a radical -CH=CH-(CH₂)r-R₈
the values p, q, r and s and the radicals Y and R₈ having the meaning given below,
- X represents the linkages of formulae (a)-(m) below which may be read in both directions: 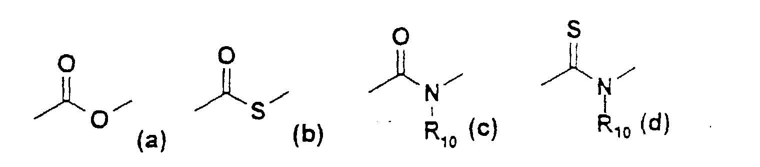 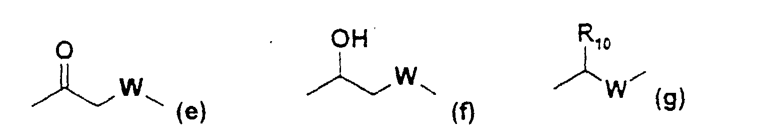 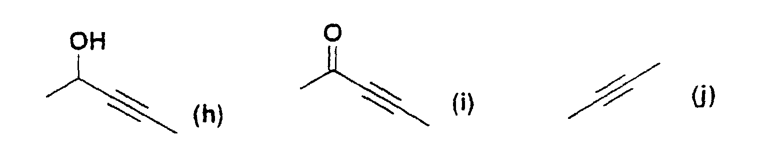 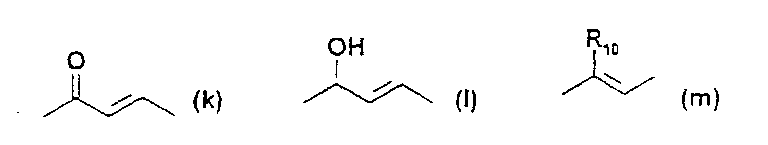
- Ar represents a radical chosen from the radicals of formulae (a)-(f) below: 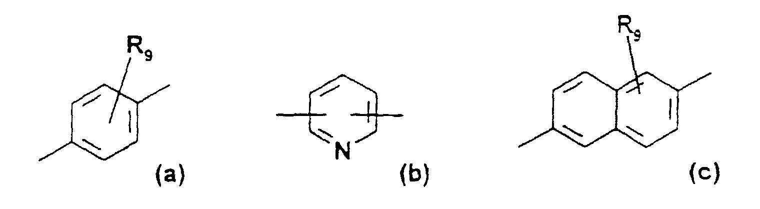 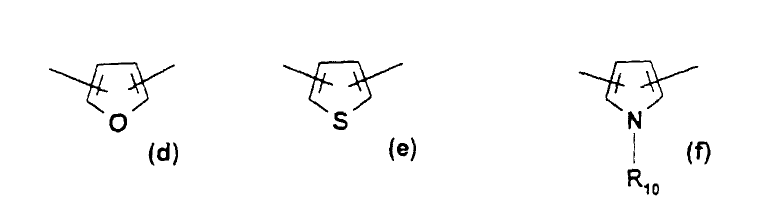 it being understood in all of the above text that:
- m is an integer equal to 1, 2 or 3
- n is an integer equal to 0 or 1
- p is an integer between 1 and 12 inclusive
- q is an integer between 0 and 12 inclusive
- r is an integer between 0 and 10 inclusive
- s is an integer equal to 0 or 1
- t is an integer equal to 0, 1 or 2
- Y represents an oxygen atom or a radical S(O)t
- W represents an oxygen atom, a radical S(O)t or a radical N-R₁₀
- R₄ represents a hydrogen atom, a lower alkyl radical or a radical -CO-R₁₁
- R₅ represents a lower alkyl or a heterocycle
- R₆ represents a hydrogen atom, a lower alkyl radical or a radical 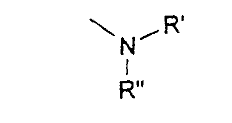 in which R' and R" represent a hydrogen atom, a lower alkyl radical, a mono- or polyhydroxyalkyl radical, an optionally substituted aryl radical or an amino acid or peptide or sugar residue, or alternatively, taken together, form a heterocycle,
- R₇ represents a hydrogen atom, a linear or branched alkyl radical having from 1 to 20 carbon atoms, an alkenyl radical, a mono- or polyhydroxyalkyl radical, an optionally substituted aryl or aralkyl radical or a sugar residue or an amino acid or peptide residue,
- R₈ represents a hydrogen atom, a branched alkyl radical having from 1 to 20 carbon atoms, a C3-C6 cycloaliphatic radical, a monohydroxyalkyl radical or a polyhydroxyalkyl radical in which the hydroxyls are optionally protected in methoxy, acetoxy or acetonide form, an aryl radical, an alkenyl radical, an alkynyl radical, a radical -CO-R₆, a radical -CO-O-R₇, an amino alkyl radical whose amine function is optionally substituted with one or two lower alkyl groups, a heterocycle,
- R₉ represents a hydrogen or halogen atom, a linear or branched alkyl radical having from 1 to 20 carbon atoms, a radical -OR₄ or an -O-CH₂-O-CH₂-CH₂-O-CH₃ radical,
- R₁₀, which may be identical or different, represents a hydrogen atom or a lower alkyl radical,
- R₁₁ represents a lower alkyl radical,
given that when X represents the linkage of formula (e) and R₃ represents the radical (iii) in which Y represents an oxygen atom and R₈ represents a hydrogen atom, then q must be greater than 6,
as well as the salts thereof and the optical and geometrical isomers thereof.

2. Compounds according to Claim 1, characterized in that they are in the form of salts of an alkali metal or alkaline-earth metal, or alternatively of zinc or of an organic amine.

3. Compounds according to either of Claims 1 and 2, characterized in that the lower alkyl radicals are chosen from the group consisting of the methyl, ethyl, isopropyl, butyl, tert-butyl, hexyl, nonyl and dodecyl radicals.

4. Compounds according to one of the preceding claims, characterized in that the linear or branched alkyl radicals having from 1 to 20 carbon atoms are chosen from the group consisting of the methyl, ethyl, propyl, 2-ethylhexyl, octyl, dodecyl, hexadecyl and octadecyl radicals.

5. Compounds according to one of the preceding claims, characterized in that the monohydroxyalkyl radicals are chosen from the group consisting of the 2-hydroxyethyl, 2-hydroxypropyl and 3-hydroxypropyl radicals.

6. Compounds according to one of the preceding claims, characterized in that the polyhydroxyalkyl radicals are chosen from the group consisting of the 2,3-dihydroxypropyl, 2,3,4-trihydroxybutyl and 2,3,4,5-tetrahydroxypentyl radicals or the pentaerythritol residue.

7. Compounds according to one of the preceding claims, characterized in that the aryl radical is a phenyl radical optionally substituted with at least one halogen atom, one hydroxyl or one nitro function.

8. Compounds according to one of the preceding claims, characterized in that the aralkyl radicals are chosen from the group consisting of the benzyl or phenethyl radicals, optionally substituted with at least one halogen atom, one hydroxyl or one nitro function.

9. Compounds according to one of the preceding claims, characterized in that the alkenyl radicals are chosen from the group consisting of radicals containing from 1 to 5 carbon atoms and having one or more ethylenic unsaturations, and in particular the allyl radical.

10. Compounds according to one of the preceding claims, characterized in that the sugar residues are chosen from the group consisting of glucose, galactose, mannose and glucuronic acid residues.

11. Compounds according to any one of the preceding claims, characterized in that the amino acid residues are chosen from the group consisting of residues derived from lysine, from glycine or from aspartic acid.

12. Compounds according to any one of the preceding claims, characterized in that the peptide residues are chosen from the group consisting of dipeptide and tripeptide residues.

13. Compounds according to any one of the preceding claims, characterized in that the heterocyclic radicals are chosen from the group consisting of the piperidino, morpholino, pyrrolidino and piperazino radicals, optionally substituted in position 4 with a C₁-C₆alkyl or mono- or polyhydroxyalkyl radical.

14. Compounds according to any one of the preceding claims, characterized in that the halogen atoms are chosen from the group consisting of fluorine, chlorine and bromine.

15. Compounds according to any one of the preceding claims, characterized in that the branched alkyl radical having from 1 to 20 carbon atoms is chosen from the 2-methylbutyl, 2-methylpentyl, 1-methylhexyl and 3-methylheptyl radicals.

16. Compounds according to any one of the preceding claims, characterized in that the aminoalkyl radical is chosen from the aminomethyl, 3-aminopropyl and 6-aminohexyl radicals.

17. Compounds according to any one of the preceding claims, characterized in that the alkynyl radical has from 2 to 6 carbon atoms.

18. Compounds according to any one of the preceding claims, characterized in that the cycloaliphatic radicals of from 3 to 6 carbon atoms are chosen from the cyclopropyl radical and the cyclohexyl radical.

19. Compounds according to Claim 1, characterized in that they are taken, alone or as mixtures, from the group consisting of:
4-[4-(1-Adamantyl)-3-methoxybenzoyloxy]benzoic acid.
(E)-4-[[2-[4-(1-Adamantyl)-3-hydroxyphenyl]-ethenyl]]benzoic acid.
(E)-4-[[2-[4-(1-Adamantyl)-3-propyloxyphenyl]-ethenyl]]benzoic acid.
(E)-4-[[2-[4-(1-Adamantyl)-3-heptyloxyphenyl]-ethenyl]]benzoic acid.
(E)-4-[[2-[4-(1-Adamantyl)-3-methoxyphenyl]-ethenyl]]benzoic acid.
4-[4-(1-Adamantyl)-3-methoxyethoxymethoxyphenylethynyl]benzoic acid.
(E)-4-[[2-[4-(1-Adamantyl)-3-(5-carbamoylpentyloxy)phenyl]ethenyl]]benzoic acid.
(E)-4-[[2-[4-(1-Adamantyl)-3-methoxyphenyl)-1-propenyl]]benzoic acid.
(E)-4-[[2-[4-(1-Adamantyl)-3-(3-hydroxypropyloxy)phenyl]ethenyl]]benzoicacid.
(E)-4-[[2-[4-(1-Adamantyl)-3-(6-hydroxyhexyloxy)phenyl]ethenyl]]benzoic acid.
4-[[3-oxo-3-[4-(1-Adamantyl)-3-methoxyphenyl)-1-propenyl]]benzoic acid.
4-[4-(1-Adamantyl)-3-methoxyethoxymethoxybenzoylthio]benzoic acid.
4-[4-(1-Adamantyl)-3-methoxyethoxymethoxybenzoyloxy]benzoic acid.
4-[4-(1-Adamantyl)-3-methoxyethoxymethoxybenzamido]benzoic acid.
(E)-4-[2-(4-(1-Adamantyl)-3-methoxyethoxymethoxyphenyl)ethenyl]benzoic acid.
(E)-4-[2-(4-(1-Adamantyl)-3-methoxyethoxymethoxyphenyl)-1-propenyl]benzoic acid.
(Z)-4-[2-(4-(1-Adamantyl)-3-methoxyethoxymethoxyphenyl)-1-propenyl]benzoic acid.
4-[4-(1-Adamantyl)-3-methoxyethoxymethoxybenzoylmethyloxy]benzoic acid.
4-[[3-Oxo-3-[4-(1-adamantyl)-3-methoxyethoxymethoxyphenyl]-1-propynyl]]benzoic acid.
4-[[3-Hydroxy-3-[4-(1-adamantyl)-3-methoxyethoxymethoxyphenyl]-1-propynyl]]benzoic acid.
(E)-4-[[3-Oxo-3-[4-(1-adamantyl)-3-methoxyethoxymethoxyphenyl)-1-propenyl]]benzoic acid.
4-[[3-Hydroxy-3-[4-(1-adamantyl)-3-methoxyethoxymethoxyphenyl)-1-propenyl]]benzoic acid.
Allyl 4-[4-(1-adamantyl)-3-methoxyethoxymethoxythiobenzamido]benzoate.
4-[[3-Hydroxy-3-[4-(1-adamantyl)-3-methoxyphenyl)-1-propynyl]]benzoic acid.
2-Hydroxy-4-[[3-hydroxy-3-[4-(1-adamantyl)-3-methoxyphenyl)-1-propynyl]]benzoic acid.
2-Hydroxy-4-[[3-hydroxy-3-[4-(1-adamantyl)-3-methoxyethoxymethoxyphenyl]]-1-propynyl]benzoic acid.
4-[[3-Hydroxy-3-[4-(1-adamantyl)-3-methoxyethoxymethoxyphenyl]-1-propynyl]]benzaldehyde.
4-[[3-Hydroxy-3-[4-(1-adamantyl)-3-methoxyethoxymethoxyphenyl]-1-propynyl]]benzenemethanol.
N-Ethyl-4-[4-(1-adamantyl)-3-methoxyethoxymethoxyphenylethynyl]benzamide.
N-(4-Hydroxyphenyl)-4-[4-(1-adamantyl)-3-methoxyethoxymethoxyphenylethynyl]benzamide.
4-[4-(1-Adamantyl)-3-methoxyethoxymethoxyphenylethynyl]phenol.

20. Compounds according to Claim 1, characterized in that they have at least one of the following characteristics:
- R₁ is the radical
-COO-R₇ and -CO-R₆
- R₃ is the radical
-Y-(CH₂)p-Y-(CH₂)q-R₈
-(CH₂)p-(Y)n-(CH₂)q-R₈
-Y-(CH₂)q-R₈
- X represents the linkage of formula (a), (h), (i), (j), (k) or (m)
- Ar represents the radical of formula (a) or (b).

21. Compounds according to any one of the preceding claims, for a use as a medicinal product.

22. Compounds according to Claim 21, for a use as a medicinal product intended for the treatment of dermatological, rheumatic, respiratory, cardiovascular and ophthalmological complaints.

23. Use of at least one of the compounds defined in Claims 1 to 20 for the manufacture of a medicinal product intended for the treatment of dermatological, rheumatic, respiratory, cardiovascular and ophthalmological complaints.

24. Pharmaceutical composition, characterized in that it comprises, in a pharmaceutically acceptable support, at least one of the compounds as defined in any one of Claims 1 to 20.

25. Composition according to Claim 24, characterized in that the concentration of compound(s) according to one of Claims 1 to 16 is between 0.001% and 5 % by weight relative to the whole composition.

26. Cosmetic composition, characterized in that it comprises, in a cosmetically acceptable support, at least one of the compounds as defined in any one of Claims 1 to 20.

27. Composition according to Claim 26, characterized in that the concentration of compound(s) according to one of Claims 1 to 20 is between 0.001% and 3 % by weight relative to the whole composition.

28. Use of a cosmetic composition as defined in either of Claims 26 and 27, for body or hair hygiene.
